# EUROPEAN PATENT APPLICATION

(11) **EP 4 700 789 A1**
(43) Date of publication of application: **25.02.2026**
(21) Application number: 24793086.0
(22) Date of filing: 22.04.2024
(51) Int. Cl.: G16H 50/20, G16B 30/00, G16B 40/20, C12Q 1/6886, G06N 3/04, G06N 3/08

(54) **METHOD FOR DIAGNOSING CANCER USING TRANSCRIPTOME-BASED IMMUNE REPERTOIRE PROFLINING**

(30) Priority: 20.04.2023 KR 20230051756
(71) Applicant: GC Genome Corporation, Yongin-si, Gyeonggi-do 16924 (KR)
(72) Inventor: KI, Chang-Seok, Yongin-si Gyeonggi-do 16924 (KR); CHO, Eun-Hae, Yongin-si Gyeonggi-do 16924 (KR); CHOI, Jung Kyoon, Daejeon 34141 (KR); SEO, Kayoung, Daejeon 34141 (KR)
(74) Representative: Mewburn Ellis LLP
(86) International application number: PCT/KR2024/005399
(87) International publication number: WO 2024/219902

(57) **Abstract**

The present invention relates to a method for diagnosing cancer using transcriptome-based immune repertoire profiling and, more particularly, to a method for diagnosing cancer by using a method of obtaining transcriptome sequence information from biological samples, then extracting, from the transcriptome sequence information, the features of immune cell receptors and the features of transcriptomes, and then analyzing same by input into an artificial intelligence model trained to determine the presence or absence of cancer. The method for diagnosing cancer using transcriptome-based immune repertoire profiling, according to the present invention, diagnoses all cancers on the basis of artificial intelligence by using both the features of immune cell receptors and the features of gene expression levels, the features being extracted from transcriptome samples generated by next generation sequencing (NGS), and is useful with high commercial applicability due to its high accuracy and sensitivity compared to diagnostic methods that use only the sequence information of immune cell receptors.

## Description

### [Technical Field]

The present invention relates to a method of diagnosing cancer using transcriptome-based immune repertoire profiling, and more specifically, to a method of diagnosing cancer including obtaining transcriptome sequence information from a biological sample, extracting features of immune cells and features of transcriptomes from the transcriptome sequence information, and inputting the features to an artificial intelligence model trained to determine the presence or absence of cancer and analyzing the characteristics.

### [Background Art]

A major goal of precision oncology is to improve the diagnosis and treatment of cancer. For this purpose, known predictive markers are identified and classification of subtypes of molecules capable of estimating prognosis is induced to select therapies using a variety of genomic and other molecular assays for a tumor material. Also, somatic changes associated with tumor progression are characterized, disrupted pathways are detected and molecular discriminators of metastatic diseases are determined. Although various next-generation sequencing (NGS)-based approaches have been used to characterize the tumor genome in detail, more accurate tumor types may be classified through comprehensive multiparameter analysis. For example, The Cancer Genome Atlas (TCGA) research network has produced comprehensive molecular profiles at the DNA, RNA, protein and epigenetic levels for hundreds of tumors. These multiparametric analyses have advanced our understanding of tumor types, the functional roles of identified new tumor subtypes and molecular variations. Importantly, these efforts have caused identification of novel drug targets, a prerequisite for realizing the promise of precision medicine. However, an approach to tumor materials for molecular profiling is not generally possible, but relies on invasive methods that are not suitable for continuous monitoring of tumor genotypes.

Thus, precision oncology has increasingly focused on liquid biopsies, which are noninvasive and allow repeated experimentation and easy monitoring of disease. In fact, attempts are being made to use these liquid biopsies for early detection of cancer. The term "liquid biopsy" was first used to describe how the same diagnostic information can be obtained from a blood sample derived from a tissue biopsy sample. In oncology, this term has been used in a broad sense to refer to the assay and sampling of various easily accessible biological fluids such as urine, ascites or pleura as well as blood.

In this case, the analyte of the body fluid peripheral blood contains circulating tumor cells (CTC), circulating cell-free DNA (cfDNA) of cancer patients containing circulating tumor DNA (ctDNA), small RNA, circulating cell-free RNA containing mRNA (cfRNA), circulating extracellular vesicles (EVs) such as exosomes, tumor educated platelets (TEPs), proteins and metabolites. In addition, these analytes have the potential to provide information about the characteristics of primary tumors or metastases commonly obtained by pathologists. In addition to information on genomic mutations and copy number alterations commonly obtained from CTCs or ctDNA, liquid biopsies are used to generate general information on transcriptomes, protoplasts, proteomes, and metabolomes (Jacob J. Chabon et al., Nature, Vol. 580, pp. 245-25, 2020).

In recent years, the results of research on designing high-performance prediction models using mutation information or epigenomic information of DNA in blood have been published, and the importance of liquid biopsy in the field of cancer diagnosis is becoming more prominent (Korean Patent Laid-open No. 2022-0072680, US Patent Laid-open No. 18/472,529).

Meanwhile, research on cancer-specific immunological aspects is actively conducted due to the development of immunotherapy for cancer.

For example, a cancer-associated TCR score as an indicator of whether or not the T cell receptor (TCR) sequence of a cancer patient is specific to cancer antigen was developed by Beshnova D. et al., Sci Transl Med. Vol.12(557), 2020. When the cancer score of a new patient in need of diagnosis is calculated by averaging the cancer-associated TCR score, which is an indicator of how many cancer-specific TCRs are present in the TCR pool of the patient, after training the amino acid sequence of the cancer-specific TCRs through a convolutional neural network, it was found that there was a difference in the distribution of cancer score between a cancer patient and a normal subject. Based on this finding, a technology useful for cancer diagnosis was developed.

In addition, the role of B cells in anticancer immune responses has recently been highlighted. For example, it has been reported that germinal center B cells, which are the core of tertiary lymphoid structures (TLS) formed in the cancer microenvironment, affect the responsiveness of immunotherapy (Helmink, B.A. et al., Nature, Vol. 577, pp. 549-555, 2020; Petitprez, F. et al., Nature, Vol. 577, pp. 556-560, 2020).

In addition, a technology has been developed to analyze the immune repertoire of patients using not only the TCR but also the complementary-determining region 3 (CDR3) sequence of the B cell receptor (BCR) from whole transcriptome sequencing (WTS) data. For example, the CDR3 sequence can be extracted from WTS data using the TRUST4 algorithm (L. Song et al., Nature Methods 18:627, 2021).

Technologies have been developed to evaluate anticancer immune responses and diagnose cancer using repertoire analysis of T cell receptors and B cell receptors. However, to date, prediction models have only been designed and evaluated with a limited number of cancer types and patients, and there are thus drawbacks such as limitations in sensitivity and accuracy.

Under this technical background, as a result of efforts to develop a cancer diagnosis method using transcriptome-based immunological profiling, the present inventors have found that cancer can be diagnosed with high sensitivity and accuracy by extracting the features of immune cells and the features of transcriptomes from the acquired transcriptome sequence information, and inputting the features to an artificial intelligence model trained to distinguish between a cancer patient and a normal subject. Based on this finding, the present invention has been completed.

### [Disclosure]

Therefore, it is one object of the present invention to provide a method of providing information for cancer diagnosis based on immunological profiling using transcriptomes.

It is another object of the present invention to provide a device and computer-readable storage medium for the method for providing information for diagnosis of cancer.

It is another object of the present invention to provide a method of diagnosing cancer based on immunological profiling using transcriptomes.

In accordance with one aspect of the present invention, the above and other objects can be accomplished by the provision of a method of providing information for cancer diagnosis based on immunological profiling using transcriptomes, the method including: (a) obtaining whole transcriptome sequencing (WTS) information from a biological sample; (b) aligning the obtained WTS information to a reference genome database; (c) extracting immune cell receptor features and transcriptome features from the aligned WTS information; and (d) inputting the extracted features to an artificial intelligence model trained to distinguish between a cancer patient and a normal subject and comparing the analyzed output value with a cut-off value to determine whether or not there is cancer.

In accordance with another aspect of the present invention, provided is a method of diagnosing cancer based on immunological profiling using transcriptomes, the method including: (a) obtaining whole transcriptome sequencing (WTS) information from a biological sample; (b) aligning the obtained WTS information to a reference genome database; (c) extracting immune cell receptor features and transcriptome features from the aligned WTS information; and (d) inputting the extracted features to an artificial intelligence model trained to distinguish between a cancer patient and a normal subject and comparing the analyzed output value with a cut-off value to determine whether or not there is cancer.

In accordance with another aspect of the present invention, provided is a device for diagnosing cancer based on immunological profiling, the device including: a decoder configured to decode whole transcriptome sequencing (WTS) information from a biological sample; an aligner configured to align the decoded WTS information to a reference genome database; a feature detector configured to extract immune cell receptor features and transcript features from the aligned WTS information; and a cancer diagnostic unit configured to compare the analyzed output obtained by inputting the extracted features to an artificial intelligence model trained to distinguish between a cancer patient and a normal subject, with a cut-off value, and determine whether or not cancer is present.

In accordance with another aspect of the present invention, provided is a computer-readable storage medium including an instruction configured to be executed by a processor for providing information for diagnosis of cancer, through the following steps including: (a) obtaining whole transcriptome sequencing (WTS) information from a biological sample; (b) aligning the obtained WTS information to a reference genome database; (c) extracting immune cell receptor features and transcriptome features from the aligned WTS information; (d) inputting the extracted features to an artificial intelligence model trained to distinguish between a cancer patient and a normal subject, and comparing the analyzed output value with a cut-off value, and primarily determining whether or not there is cancer; and (e) inputting the immune cell receptor features and transcriptome features of the sample primarily determined that there is cancer into a second artificial intelligence model trained to distinguish between an immunoactive state and cancer, comparing the analyzed output value with a cut-off value and finally determining whether or not there is cancer.

### [Description of Drawings]

FIG. 1 is an overall flowchart illustrating a method of diagnosing cancer based on immunological profiling using transcriptomes according to the present invention, and shows the composition of data used to train the artificial intelligence model and the types of input values.
FIG. 2 is a conceptual diagram illustrating an input matrix input to an artificial intelligence model constructed according to an embodiment of the present invention.
FIG. 3 shows the performance of a DNN model trained for use in immunological profiling-based cancer diagnosis using transcriptomes constructed according to an embodiment of the present invention, where (A) shows the result of various evaluation indices of the model and (B) shows the distribution of output values derived from the trained model.
FIG. 4 shows the performance of a linear regression model and a random forest model trained for use in cancer diagnosis based on immunological profiling using transcriptomes constructed according to an embodiment of the present invention.
FIG. 5 shows the importance of each feature in a DNN model constructed according to an embodiment of the present invention.
FIG. 6 shows the confusion matrix to identify false positives and false negatives of the DNN model constructed according to an embodiment of the present invention.
FIG. 7 is a flowchart illustrating construction of a diagnostic model for performing false positive filtering to optimize the DNN model constructed according to an embodiment of the present invention.
FIG. 8 shows the result of comparison in the performance of the optimized DNN model constructed according to an embodiment of the present invention with a DNN model before optimization, wherein PR-AUC represents the overall performance of precision and reproduction, ROC-AUC represents the ability to distinguish positive/negative, and accuracy is an indicator of whether or not the prediction was correct. PR-AUC is useful for evaluation of the performance of a model in unbalanced data.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

Terms such as first, second, A, and B may be used to describe various elements, but these elements are not limited by these terms and are merely used to distinguish one element from another. For example, without departing from the scope of the technology described below, a first element may be referred to as a second element and in a similar way, the second element may be referred to as a first element. "And/or" includes any combination of a plurality of related recited items or any one of a plurality of related recited items.

Singular forms are intended to include plural forms as well, unless the context clearly indicates otherwise. It will be further understood that the terms "comprises" and/or "comprising", when used in this specification, specify the presence of features, numbers, steps, actions, components, parts, or combinations thereof, but do not preclude the presence or addition of one or more other features, numbers, steps, actions, components, parts, or combinations thereof.

Prior to the detailed description of the drawings, it is to be clarified that the classification of components in the present specification is merely made depending on the main function of each component. That is, two or more components described below may be combined into one component or one component may be divided into two or more depending on each more detailed function. In addition, each component to be described below may further perform some or all of the functions of other components in addition to its main function, and some of the main functions of each component may be performed exclusively by other components.

In addition, in implementing a method or operation method, respective steps constituting the method may occur in a different order from a specific order unless the specific order is clearly described in context. That is, the steps may be performed in the specific order, substantially simultaneously, or in reverse order to that specified.

The present invention is intended to determine whether or not cancer can be detected with high sensitivity and accuracy by aligning whole transcriptome sequencing (WTS) data obtained from a sample with a reference genome database, extracting immunological features and transcriptomic features from the aligned WTS information, and inputting the features to an artificial intelligence model trained to distinguish a cancer patient from a normal subject.

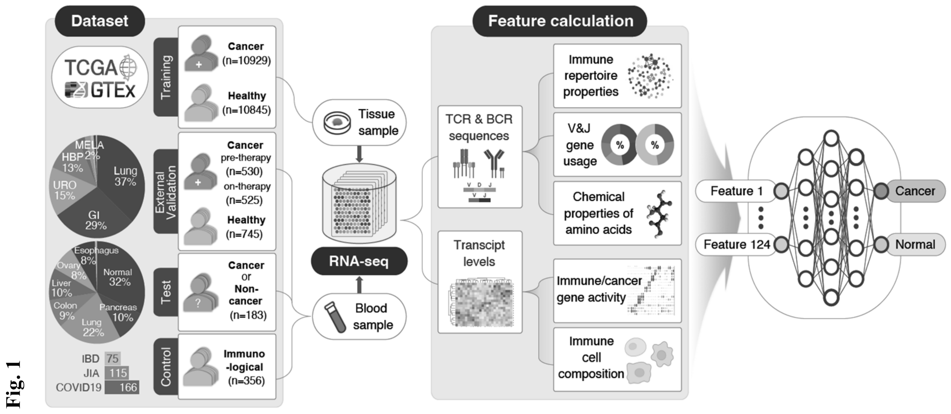

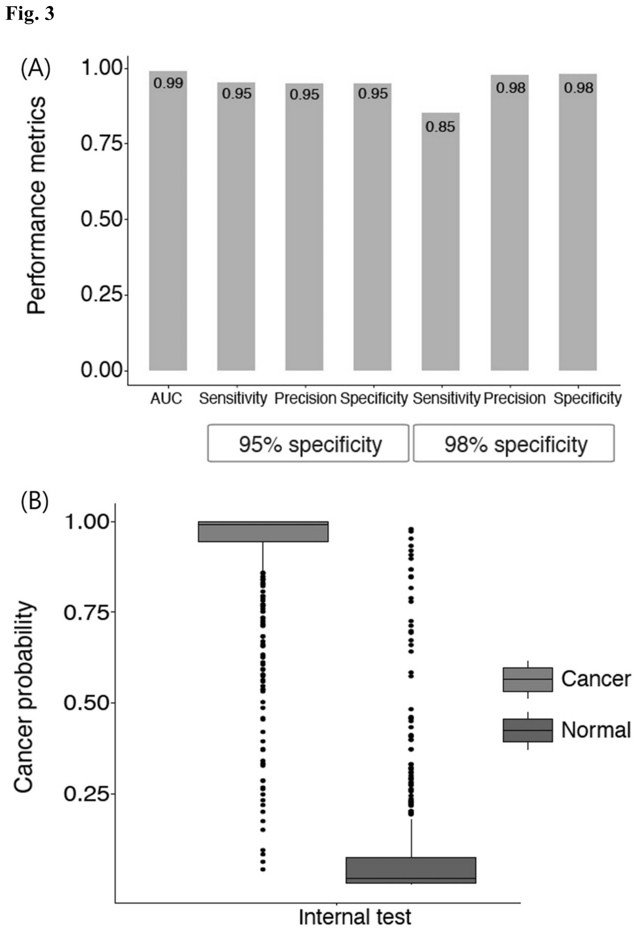

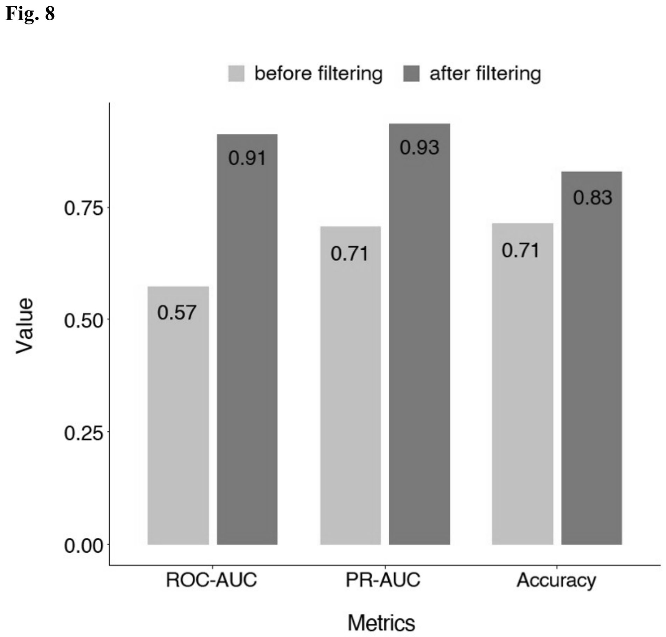

That is, in one embodiment of the present invention, a training model capable of diagnosing cancer was constructed using 124 features including immunological features and transcriptome features from the WTS data (FIG. 1). The performance was tested using WTS data of 183 cancer patients. The result showed that the training model had excellent performance (FIGS. 3 and 8).

As used herein, the term "read" refers to a single nucleic acid fragment, sequence information of which is analyzed using various methods known in the art. Therefore, the terms "sequence information", "transcriptome sequencing information", and "read" have the same meaning in that all are sequence information obtained through a sequencing process.

In one aspect,
the present invention is directed to a method of providing information for cancer diagnosis based on immunological profiling using transcriptomes, the method including:
(a) obtaining whole transcriptome sequencing (WTS) information from a biological sample;
(b) aligning the obtained WTS information to a reference genome database;
(c) extracting immune cell receptor features and transcriptome features from the aligned WTS information; and
(d) inputting the extracted features to an artificial intelligence model trained to distinguish between a cancer patient and a normal subject and comparing the analyzed output value with a cut-off value to determine whether or not there is cancer.

In another aspect,
the present invention is directed to a method of diagnosing cancer based on immunological profiling using transcriptomes, the method including:
(a) obtaining whole transcriptome sequencing (WTS) information from a biological sample;
(b) aligning the obtained WTS information to a reference genome database;
(c) extracting immune cell receptor features and transcriptome features from the aligned WTS information; and
(d) inputting the extracted features to an artificial intelligence model trained to distinguish between a cancer patient and a normal subject and comparing the analyzed output value with a cut-off value to determine whether or not there is cancer.

In the present invention, the immune cell receptor feature may be used without limitation as long as it is a feature of an immune-related cell receptor that can be obtained from WTS information, and preferably, the immune cell receptor feature may be a feature of T cell receptors (TCR), or a feature of B cell receptors (BCR), based on the CDR3 (complementary-determining region 3) amino acid sequence derived from WTS information, or a feature of the CDR3 amino acid sequence, but is not limited thereto.

In the present invention, any transcriptome feature may be used without limitation as long as it is a transcriptome-related feature that may be obtained from WTS information, and preferably, the transcriptome feature is an enrichment score or an immune cell composition of a cancer- or immune response-related gene, but is not limited thereto.

In the present invention, the obtaining whole transcriptome sequencing (WTS) information from the biological sample in step (a) may include the following steps:
(a-i) randomly fragmenting RNA obtained from a biological sample using an enzymatic cleavage method;
(a-ii) replacing the cleavage RNA with cDNA and then producing a library; and
(a-iii) performing single-end sequencing or pair-end sequencing on the produced library using a next-generation sequencer to obtain WTS information.

In the present invention, the obtaining WTS information in step (a) may include obtaining WTS information from the obtained cDNA through whole transcriptome sequencing at a read depth of 1 million to 150 million, preferably at a read depth of 5 million to 100 million, and most preferably at an average read depth of 90 million, but is not limited thereto.

In the present invention, the biological sample refers to any substance, biological fluid, tissue or cell obtained from or derived from a subject, and examples thereof include, but are not limited to, whole blood, leukocytes, peripheral blood mononuclear cells, leukocyte buffy coat, blood including plasma and serum, sputum, tears, mucus, nasal washes, nasal aspirates, breath, urine, semen, saliva, peritoneal washings, pelvic fluids, cystic fluids, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, semen, hair, saliva, urine, oral cells, placenta cells, cerebrospinal fluid, and mixtures thereof.

As used herein, the term "reference population" refers to a reference group that is used for comparison like a reference genome database and refers to a population of subjects who do not currently have a specific disease or condition. In the present invention, the reference nucleotide sequence in the reference genome database of the reference population may be a reference chromosome registered with public health institutions such as the NCBI.

In the present invention, the RNA in step (a) may be mRNA, but is not limited thereto.

In the present invention, the next-generation sequencer may be used for any sequencing method known in the art. Sequencing of nucleic acids isolated using the selection method is typically performed using next-generation sequencing (NGS). Next-generation sequencing includes any sequencing method that determines the nucleotide sequence either of each nucleic acid molecule or of a proxy cloned from each nucleic acid molecule so as to be highly similar thereto (e.g., 105 or more molecules are sequenced simultaneously). In one embodiment, the relative abundance of nucleic acid species in the library can be estimated by counting the relative number of occurrences of the sequence homologous thereto in data produced by sequencing experimentation. Next-generation sequencing is known in the art, and is described, for example, in Metzker, M. (2010), Nature Biotechnology Reviews 11:31-46, which is incorporated herein by reference.

In one embodiment, next-generation sequencing is performed to determine the nucleotide sequence of each nucleic acid molecule (using, for example, a HelioScope Gene-Sequencing system from Helicos Biosciences or a PacBio RS system from Pacific Biosciences). In other embodiments, massive parallel short-read sequencing, which produces more bases of the sequence per sequencing unit than other sequencing methods, for example, other sequencing methods that produce fewer but longer reads, determines the nucleotide sequence of a proxy cloned from each nucleic acid molecule (using, for example, a Solexa sequencer from Illumina Inc., located in San Diego, CA; 454 Life Sciences (Branford, Connecticut) and Ion Torrent). Other methods or devices for next-generation sequencing may be provided by 454 Life Sciences (Branford, Connecticut), Applied Biosystems (Foster City, CA; SOLiD Sequencer), Helicos Biosciences Corporation (Cambridge, MA) and emulsion and microfluidic sequencing nanodrops (e.g., GnuBIO Drops), but are not limited thereto.

Platforms for next-generation sequencing include, but are not limited to, the FLX System genome sequencer (GS) from Roche/454, the Illumina/Solexa genome analyzer (GA), the Support Oligonucleotide Ligation Detection (SOLiD) system from Life/APG, the G.007 system from Polonator, the HelioScope gene-sequencing system from Helicos Biosciences, and the PacBio RS system from Pacific Biosciences.

In the present invention, the alignment of step (b) may be performed using the STAR algorithm and the Hgl9 sequence, but is not limited thereto.

The parameters of the STAR algorithm used herein have the following values, but are not limited thereto:
--outFilterMismatchNoverLmax 0.05: output only when the mismatch ratio of the mapped length is lower than this value.
--outFilterMatchNmin 20: output only when the number of matching bases is greater than this value.
--alignIntronMax 0: the maximum intron size is set to 0.

In the present invention, the WTS information obtained after the alignment of (b) may be in a bam file format, but is not limited thereto.

In the present invention, the step (c) of extracting the immune cell receptor features may include the following steps:
(c-i) extracting a CDR3 (complementary-determining region 3) amino acid sequence of the immune cell receptor from the aligned WTS information; and
(c-ii) calculating at least one feature selected from the group consisting of immune repertoire properties, V and J gene usage, and chemical features of the CDR3 amino acid sequence based on the extracted sequence information.

In the present invention, the immune repertoire properties include at least one selected from the group consisting of the total number of TCR sequences, the unique number of TCR sequences, the number of TCR clonotypes per kilo-reads, the entropy of TCR, the clonality of TCR, the total number of BCR sequences, the unique number of BCR sequences, the number of BCR clonotypes per kilo-reads, the entropy of BCR, the clonality of BCR, the somatic hypermutation rate of BCR sequences, and the number of BCR clusters.

In the present invention, the V&J gene usage may be a genotype-specific proportion of the CDR3 sequence of each of V and J regions for each sample, and may be a proportion of two or more genotypes selected from the group consisting of IGH, IGK, IGL, TRA, TRB, TRD, TRG, IGHA1, IGHA2, IGHD, IGHE, IGHG1, IGHG2, IGHG3, IGHG4, IGHGP, and IGHM, and may be most preferably a proportion of each of the 17 genotypes using the following Equation 2:

$\begin{matrix}gene usage of genotype i \\ = \frac{number of CDR 3 sequences corresponding to genotype i in the V & J regions}{total number of CDR 3 sequences}\end{matrix}$

In the present invention, the chemical properties of the CDR3 amino acid sequence include at least one selected from the group consisting of the mean of length in TCR, the mean of gravy index in TCR, the mean of bulkiness of amino acids in TCR, the mean of polarity of amino acids in TCR, the mean of net charge in TCR, the mean of fraction of basic amino acids in TCR, the mean of fraction of acidic amino acids in TCR, the mean of fraction of aromatic amino acids in TCR, the mean of aliphatic index in TCR, the mean of length in BCR, the mean of gravy index in BCR, the mean of bulkiness of amino acids in BCR, the mean of BCR amino acids, the mean of polarity of amino acids in BCR, the mean of net charge in BCR, the mean of fraction of basic amino acids in BCR, the mean of fraction of acidic amino acids in BCR, the mean of fraction of aromatic amino acids in BCR, and the mean of aliphatic index in BCR.

In the present invention, the step (c) of extracting transcriptome features may include calculating a cancer- or immune-related gene enrichment score (single sample gene set enrichment analysis, ssGESA score) or an immune cell composition.

In the present invention, the cancer- or immune-related gene enrichment score (single sample gene set enrichment analysis, ssGESA score) is obtained by calculating an enrichment score of a gene associated with at least one selected from the group consisting of T cell activation involved in immune response, innate immune response, innate immune response activating cell surface receptor signaling pathway, lymphocyte activation involved in immune response, mucosal immune response, natural killer cell activation involved in immune response, negative regulation of immune response, negative regulation of innate immune response, neutrophil activation involved in immune response, positive regulation of cytokine, production involved in immune response, positive regulation of immune effector process, positive regulation of immune response, positive regulation of production of molecular mediator of immune response, regulation of adaptive immune response, regulation of cytokine production involved in immune response, regulation of humoral immune response, regulation of immune effector process, regulation of immune response, regulation of innate immune response, antigen processing and presentation, B cell receptor signaling pathway, chemokine signaling pathway, complement and coagulation cascades, cytosolic DNA-sensing pathway, Fc epsilon RI signaling pathway, Fc gamma R-mediated phagocytosis, hematopoietic cell lineage, IL-17 signaling pathway, intestinal immune network for IgA production, leukocyte transendothelial migration, NOD-like receptor signaling pathway, natural killer cell mediated cytotoxicity, neutrophil extracellular trap formation, platelet activation, RIG-I-like receptor signaling pathway, T cell receptor signaling pathway, Th1 and Th2 cell differentiation, toll-like receptor signaling pathway, cellular response to tumor necrosis factor, negative regulation of tumor necrosis factor production, negative regulation of tumor necrosis factor superfamily cytokine production, negative regulation of tumor necrosis factor-mediated signaling pathway, positive regulation of tumor necrosis factor production, positive regulation of tumor necrosis factor superfamily cytokine production, regulation of tumor necrosis factor production, regulation of tumor necrosis factor-mediated signaling pathway, response to tumor necrosis factor, tumor necrosis factor-mediated signaling pathway, central carbon metabolism in cancer, endometrial cancer, PD-L1 expression and PD-1 checkpoint pathway in cancer, and transcriptional misregulation in cancer.

In the present invention, any gene may be used without limitation as long as it is a gene involved in the pathway, and preferably, the gene may include at least one selected from the group of genes for each pathway set in Table 5 below, and most preferably, the gene may be calculated using all of the genes described in Table 5 below, but is not limited thereto.

In the present invention, the expression "at least one selected from the group of genes for each pathway set in Table 5" means, for example, when the gene is involved in the negative regulation of tumor necrosis factor-mediated signaling pathway, the gene includes at least one selected from the group consisting of ADIPOQ, NLRP2B, GSTP1, CARDS, APOA1, F2RL1, PYDC2, CCDC3, PTPN2, GPS2, PELI3, TRAIP, H2BC11, PYDC1, CARD16, GAS6, ZNF675 and RFFL.

**[Table 5]**

| Cellular response to tumor necrosis factor (GO:0071356) | | | | | | | |
|---|---|---|---|---|---|---|---|
| ACTN4 | AKT1 | ZC3H12A | TNFRSF11B | PSMB7 | ZFAND6 | TNFRSF4 | TNFRSF18 |
| ACOD1 | PSMD1 | OCSTAMP | CX3CL1 | SMPD4 | ZFP36L1 | EIF5A | TNFRSF14 |
| PSMB9 | PSMD2 | INPPSK | ANKRD1 | IKBKB | TNF | XCL1 | CHUK |
| PSMC6 | GBP1 | NKX3-1 | RIPK1 | TMSB4X | CIB1 | TNFRSF 1A | AIM2 |
| PSMC4 | GBP3 | TNFSF11 | STAT1 | PSMB8 | FOXO3 | TNFRSF 1B | LTBR |
| PSMC5 | GBP2 | TNFSF1 2 | ERBIN | PSMBS | TANK | NFKBIA | LTB |
| PSMC2 | ST18 | PSMF1 | DAB2IP | PSMB6 | ZFP36L2 | TNFRSF 21 | CDIP1 |
| TNFSF4 | EDARADD | CCL26 | PSMA6 | HYAL1 | PYCARD | PYDC1 | LTA |
| PSMC3 | TNFSF1 8 | GSDME | PSMA7 | PSMB3 | ZFP36 | XCL2 | YBX3 |
| PSMC1 | TNFSF1 5 | CCL25 | PSMA4 | HYAL2 | HAS2 | TNFRSF 25 | TNFSF1 3B |
| TNFSF9 | TNFSF1 3 | CCL24 | PSMA5 | PSMB4 | RPS3 | CHI3L1 | CCL8 |
| TNFSF8 | TNFSF1 4 | CCL23 | ADAM17 | PSMB1 | TRAF1 | NFE2L2 | CCL7 |
| TP53 | NR1D1 | CCL22 | PSMA2 | HYAL3 | TRAF3 | EDA | CCL5 |
| SFRP1 | COMMD7 | TNFRSF 12A | PSMA3 | PSMB2 | TRAF2 | PSMD11 | CCL4 |
| ASAH1 | PSME4 | CCL21 | PSMA1 | CCL19 | TRAF5 | CXCL8 | PTK2B |
| PSMB11 | PSME2 | CCL20 | CACTIN | CD58 | TRAF6 | PSMD10 | CCL3 |
| PSMB10 | PSME3 | SIRT1 | CD40LG | CCL18 | MAP3K1 4 | PSMD13 | CCL2 |
| PSMD9 | PSME1 | DCSTAM P | CCL15 | CCL17 | CARD14 | PSMD12 | CCL1 |
| CRHBP | TDGF1 | ARHGEF 2 | CCL14 | JAK2 | BIRC2 | CCL3L1 | ADAMTS 7 |
| PSMD7 | PID1 | CD27 | CCL13 | CCL16 | BIRC3 | PSMD14 | |
| PSMD8 | TNFRSF 6B | LIMS1 | GBA | NPNT | CCL4L1 | ILK | |
| PSMD5 | GATA3 | CD40 | CCL11 | CD70 | HMHB1 | TRADD | |
| PSMD6 | EDA2R | TNFRSF 11A | TNFRSF 13C | EDAR | BAG4 | ADAMTS 12 | |
| PSMD3 | TXNDC1 7 | DHX9 | TNFRSF 13B | RELA | TNFRSF 8 | GPER1 | |
| PSMD4 | TCL1A | BRCA1 | PSMA8 | PLVAP | TNFRSF 9 | TNFRSF 17 | |

| Negative regulation of tumor necrosis factor production (GO:0032720) | | | | | | | |
|---|---|---|---|---|---|---|---|
| ADIPOQ | LILRB1 | POMC | BPI | SLAMF1 | PTPN22 | ZC3H12 A | LGALS9 |
| IL10 | NR1H4 | MC1R | GHRL | ORM1 | LILRA4 | IRAK3 | CD34 |
| IL37 | DEFB11 4 | SELENO S | CACTIN | CHRNA7 | CX3CL1 | SIRPA | GHSR |
| ARG2 | IGF1 | SYT11 | PTPN6 | GSTP1 | NLRC3 | LBP | CD33 |
| NFKBIL 1 | CIDEA | CLEC4A | GAS6 | C5AR2 | VSIR | TRIM27 | |
| DICER1 | IL4 | RARA | TLR4 | TNFAIP 3 | ILRUN | HAVCR2 | |

| negative regulation of tumor necrosis factor superfamily cytokine production (GO:1903556) | | | | | | | |
|---|---|---|---|---|---|---|---|
| ADIPOQ | LILRB1 | POMC | BPI | TLR4 | C5AR2 | VSIR | TRIM27 |
| IL10 | NR1H4 | MC1R | GHRL | SLAMF1 | TNFAIP 3 | ILRUN | HAVCR2 |
| IL37 | DEFB11 4 | SELENO S | CACTIN | CD274 | PTPN22 | ZC3H12 A | LGALS9 |
| ARG2 | IGF1 | SYT11 | LTF | ORM1 | LILRA4 | IRAK3 | CD34 |
| NFKBIL 1 | CIDEA | CLEC4A | PTPN6 | CHRNA7 | CX3CL1 | SIRPA | GHSR |
| DICER1 | IL4 | RARA | GAS6 | GSTP1 | NLRC3 | LBP | CD33 |

| Negative regulation of tumor necrosis factor-mediated signaling pathway (GO:0010804) | | | | | | | |
|---|---|---|---|---|---|---|---|
| ADIPOQ | NLRP2B | GSTP1 | CARD8 | APOA1 | F2RL1 | PYDC2 | CCDC3 |
| PTPN2 | GPS2 | PELI3 | TRAIP | H2BC11 | PYDC1 | CARD16 | GAS6 |
| ZNF675 | RFFL | | | | | | |

| positive regulation of tumor necrosis factor production (GO:0032760) | | | | | | | |
|---|---|---|---|---|---|---|---|
| ARFGEF 2 | IL1A | TLR9 | NOD2 | HAVCR2 | HLA-E | TWIST1 | IL12B |
| CCR2 | TLR2 | ARHGEF 2 | CLU | IL33 | IL17A | LILRA2 | PLCG2 |
| MMP8 | CD2 | PF4 | PTPRJ | DDX58 | MAVS | AGER | CD14 |
| SPHK2 | TLR1 | TLR4 | HSPB1 | IFNGR1 | FRMD8 | LILRA5 | CCL19 |
| WNT5A | PTPRC | TLR3 | PYCARD | FZD5 | ORM2 | IFIH1 | LGALS9 |
| PTPN11 | IL6 | TMEM10 6A | SPN | SYK | ORM1 | C1QTNF 4 | EPHB2 |
| AZU1 | OAS2 | APP | CLEC7A | STAT3 | SETD4 | DDT | JAK2 |
| OAS1 | IL23A | DHX9 | LBP | MIF | LPL | RASGRP 1 | |
| TYROBP | OAS3 | LY96 | FADD | TIRAP | THBS1 | TNFRSF 8 | |
| ISL1 | IFNG | HMGB1 | RIPK1 | CYBA | PSEN1 | CCL3 | |

| Positive regulation of tumor necrosis factor superfamily cytokine production (GO:1903557) | | | | | | | |
|---|---|---|---|---|---|---|---|
| ARFGEF 2 | IL1A | TLR9 | LY96 | FADD | TIRAP | LPL | DDT |
| CCR2 | TLR2 | ARHGEF 2 | HMGB1 | RIPK1 | CYBA | BCL10 | RASGRP 1 |
| MMP8 | CD2 | ADAM8 | NOD2 | HAVCR2 | HLA-E | THBS1 | TNFRSF 8 |
| SPHK2 | TLR1 | PF4 | CLU | IL33 | IL17A | PSEN1 | CCL3 |
| WNT5A | PTPRC | TLR4 | PTPRJ | DDX58 | MAVS | TWIST1 | IL12B |
| PTPN11 | IL6 | TLR3 | HSPB1 | IFNGR1 | FRMD8 | LILRA2 | PLCG2 |
| AZU1 | OAS2 | TMEM10 6A | PYCARD | FZD5 | IL17F | AGER | CD14 |
| OAS1 | IL23A | APP | SPN | SYK | ORM2 | LILRA5 | CCL19 |
| TYROBP | OAS3 | CD86 | CLEC7A | STAT3 | ORM1 | IFIH1 | LGALS9 |
| ISL1 | IFNG | DHX9 | LBP | MIF | SETD4 | C1QTNF 4 | EPHB2 |
| JAK2 | | | | | | | |

| Regulation of tumor necrosis factor production (GO:0032680) | | | | | | | |
|---|---|---|---|---|---|---|---|
| WNT5A | NOD2 | C5AR2 | MMP8 | ARHGEF 2 | SPN | NFKBIL 1 | THBS1 |
| DICER1 | CLU | PSEN1 | SPHK2 | RARA | CLEC7A | NR1H4 | LILRA2 |
| CIDEA | PYCARD | TWIST1 | LILRB1 | GAS6 | IRAK3 | DEFB11 4 | LILRA4 |
| TYROBP | ZFP36 | AGER | PTPN11 | TLR4 | LBP | IL17A | VSIR |
| ISL1 | GHSR | C1QTNF 4 | AZU1 | TLR3 | FADD | POMC | LILRA5 |
| FOXP1 | DDX58 | RASGRP 1 | OAS1 | TMEM10 6A | RIPK1 | SYT11 | IFIH1 |
| CD2 | FZD5 | ZC3H12 A | IL1A | DHX9 | HAVCR2 | BPI | DDT |
| IL23A | MIF | SIRPA | TLR2 | TNFAIP 3 | CD34 | GHRL | CCL3 |
| SELENO S | TIRAP | TNFRSF 8 | TLR1 | PTPN22 | CD33 | CACTIN | TRIM27 |
| MAPKAP K2 | IGF1 | IL12B | IL4 | LY96 | ADIPOQ | CD47 | CCL19 |
| CLEC4A | CYBA | PLCG2 | PTPRC | HMGB1 | IL33 | LTF | EPHB2 |
| PF4 | HLA-E | CD14 | IL6 | PTPRJ | IFNGR1 | ORM2 | JAK2 |
| PTPN6 | MC1R | LGALS9 | OAS2 | CX3CL1 | IL37 | ORM1 | |
| SLAMF1 | MAVS | ARFGEF 2 | OAS3 | NLRC3 | SYK | SETD4 | |
| APP | FRMD8 | CCR2 | IFNG | HSPB1 | ARG2 | CX3CR1 | |
| CHRNA7 | LPL | IL10 | TLR9 | ILRUN | STAT3 | GSTP1 | |

| Regulation of tumor necrosis factor-mediated signaling pathway (GO:0010803) | | | | | | | |
|---|---|---|---|---|---|---|---|
| PTPN2 | MADD | TAX1BP 1 | HSPA1B | RNF31 | CASP1 | LAPTM5 | BIRC2 |
| NLRP2B | CYLD | PYDC2 | PRKN | PYCARD | RIPK1 | TRAF2 | BIRC3 |
| SPHK1 | APOA1 | RBCK1 | GPS2 | CASP8 | RFFL | TRAIP | SPPL2B |
| CFLAR | OTULIN | CCDC3 | TRADD | SHARPI N | ADIPOQ | CLIP3 | GSTP1 |
| CARD8 | H2BC11 | UBE2K | SPATA2 | CASP4 | SYK | ADAM17 | PELI3 |
| HIPK1 | HSPA1A | GAS6 | TNFAIP 3 | CPNE1 | CHUK | F2RL1 | SPPL2A |
| TNFRSF 1A | PYDC1 | ZNF675 | TNF | FADD | TRAF1 | CARD16 | IKBKB |
| RACK1 | IKBKG | | | | | | |

| Response to tumor necrosis factor (GO:0034612) | | | | | | | |
|---|---|---|---|---|---|---|---|
| NPNT | HAS2 | NUB1 | CCL23 | CCL3L1 | IGBP1 | SMPD4 | CCL18 |
| ACOD1 | AKT1 | TCL1A | CCL22 | TNFRSF 11A | MAP4K3 | IKBKB | CCL17 |
| ADAM9 | GBP1 | ZC3H12 A | CCL21 | DHX9 | GCH1 | HYAL1 | ADAMTS 7 |
| RELA | RPS3 | BAG4 | CCL20 | YTHDC2 | CHUK | CCL8 | JAK2 |
| ZFAND6 | GBP3 | UBD | XCL1 | TRADD | ADAM10 | HYAL2 | CCL16 |
| ZFP36L 1 | GBP2 | OCSTAM P | SPHK1 | BRCA1 | ERBIN | CCL7 | |
| CIB1 | GGT5 | INPP5K | SIRT1 | CX3CL1 | DAB2IP | HYAL3 | |
| SFRP1 | GGT7 | NKX3-1 | TNFRSF 21 | ADAMTS 12 | CACTIN | SMPD1 | |
| ASAH1 | NR1D1 | GGT2 | DCSTAM P | AFF3 | TRIM32 | CCL5 | |
| TANK | SELE | GGT1 | XCL2 | CASP8 | CCL15 | CCL4 | |
| CXCL16 | TDGF1 | MAP2K7 | ARHGEF 2 | GPER1 | CCL14 | CCL3 | |
| ZFP36L 2 | PID1 | CCL26 | CHI3L1 | CASP3 | CCL13 | CCL2 | |
| CRHBP | CCL4L1 | GSDME | NFE2L2 | ANKRD1 | GBA | CCL1 | |
| PYCARD | HMHB1 | CCL25 | CXCL8 | RIPK1 | CCL11 | CCL19 | |
| ZFP36 | GATA3 | CCL24 | CD40 | ADIPOQ | YBX3 | CD58 | |

| Tumor necrosis factor-mediated signaling pathway (GO:0033209) | | | | | | | |
|---|---|---|---|---|---|---|---|
| ACTN4 | RELA | PSMD1 | TRAF6 | TNFRSF 9 | PSMD11 | STAT1 | LTA |
| CD70 | PLVAP | PSMD2 | PSME2 | TNFSF1 2 | PSMD10 | CHUK | CD40LG |
| PSMB9 | TNF | ST18 | PSME3 | PSMF1 | PSMD13 | PSMA6 | TNFRSF 13C |
| EDAR | FOXO3 | EDARAD D | PSME1 | TNFRSF 4 | CD40 | PSMA7 | TNFRSF 13B |
| PSMC6 | PSMB11 | TNFSF1 8 | MAP3K1 4 | EIF5A | PSMD12 | AIM2 | TNFSF1 3B |
| PSMC4 | PSMB10 | TNFSF1 5 | CARD14 | TNFRSF 12A | TNFRSF 11A | PSMA4 | PSMA8 |
| PSMC5 | PSMD9 | TNFSF1 3 | BIRC2 | TNFRSF 1A | PSMD14 | PSMA5 | PSMB7 |
| PSMC2 | PYCARD | TNFSF1 4 | BIRC3 | TNFRSF 1B | ILK | ADAM17 | IKBKB |
| TNFSF4 | PSMD7 | TRAF1 | TNFRSF 6B | NFKBIA | TRADD | LTBR | TMSB4X |
| PSMC3 | PSMD8 | TRAF3 | EDA2R | PYDC1 | TNFRSF 11B | PSMA2 | PSMB8 |
| PSMC1 | PSMD5 | TRAF2 | TXNDC1 7 | CD27 | TNFRSF 17 | PSMA3 | PSMB5 |
| TNFSF9 | PSMD6 | COMMD7 | BAG4 | LIMS1 | TNFRSF 18 | PSMA1 | PSMB6 |
| TNFSF8 | PSMD3 | TRAF5 | TNFRSF 8 | TNFRSF 25 | RIPK1 | LTB | PSMB3 |
| TP53 | PSMD4 | PSME4 | TNFSF1 1 | EDA | TNFRSF 14 | CDIP1 | PSMB4 |
| PSMB1 | PSMB2 | PTK2B | JAK2 | | | | |

| T cell activation involved in immune response (GO:0002286) | | | | | | | |
|---|---|---|---|---|---|---|---|
| IFNA14 | SLC11A 1 | IFNW1 | IFNK | IFNA8 | IFNA4 | MDK | |
| CD74 | IFNA17 | IFNE | IFNA10 | IFNA7 | ITGAL | IFNB1 | |
| TNFSF1 8 | EIF2AK 4 | LCP1 | IFNA6 | IFNA2 | CD1C | FCER1G | |
| IFNA16 | LILRB1 | F2RL1 | IFNA5 | IFNA1 | ICAM1 | IFNA21 | |

| Innate immune response (GO:0045087) | | | | | | | |
|---|---|---|---|---|---|---|---|
| KRT1 | APOBEC 3G | LYN | ZDHHC1 1 | DEFA1 | CRISP3 | TRIM51 | SHFL |
| CHGA | KIR2DS 1 | DEFB10 4A | STING1 | TYROBP | FCN3 | DDX3X | TRIM34 |
| MX1 | KIR2DS 2 | SIGLEC 16 | RNASE3 | MID2 | CALCOC O2 | TRIM64 C | TRIM35 |
| PML | SLC11A 1 | SIGLEC 15 | DEFB10 6A | ANKHD1 | ANKRD1 7 | SHMT2 | NRROS |
| POLR3A | KIR2DS 4 | SIGLEC 14 | RNASE2 | CXCL10 | NCF2 | HMGB1 | CRP |
| IFI27 | KIR2DS 5 | CARDS | HCK | CLEC4D | PHB | NLRC5 | COLEC1 2 |
| OTULIN | PTK6 | SFTPD | IGLL5 | CLEC4E | CORO1A | TREM1 | SRC |
| SLPI | SRPK1 | TLR2 | IGLL1 | CAPZA2 | AGER | ADAR | COLEC1 1 |
| TRIM14 | HLA-E | NCR2 | ADAM15 | CD300E | CLEC5A | TREM2 | KIR3DS 1 |
| TRIM15 | SRPK2 | BST2 | TTC4 | S100A7 | UBD | SIRPB1 | COLEC1 0 |
| TRIM17 | PTK2 | CYLD | PPARG | CAPZA1 | TNK1 | INAVA | TRIM31 |
| TRIM10 | BPIFB1 | KRT16 | TRIML1 | KLRD1 | PI3 | TKFC | PLA2G1 B |
| ALPK1 | TREML1 | CFHR5 | SAA1 | NCF1 | KLRC2 | CD300L B | CX3CR1 |
| TRIM11 | VNN1 | TLR9 | TRIML2 | RNF187 | TRIM68 | IRAK1 | PIK3CD |
| TRIM13 | BPIFB3 | REL | MSRB1 | FRK | TRIM61 | LBP | PIK3CG |
| WFDC13 | BTK | TLR8 | APCS | FCN1 | TRIM62 | TRIM48 | MALT1 |
| AKAP1 | WFDC10 B | TLR4 | PGLYRP 4 | RELA | TRIM64 | TRIM49 | H2BC21 |
| APP | WFDC10 A | TLR3 | PGLYRP 3 | MASP1 | TRIM60 | TRIM43 | DHX58 |
| CYBC1 | APOBEC 3A | CD40 | ZDHHC1 | SLA | IFNLR1 | CIITA | KYNU |
| WFDC12 | CFB | CITED1 | DEFB12 7 | RNF39 | IRAK4 | CR1 | TRIM25 |
| WFDC11 | WFDC9 | MEFV | IFIH1 | IFI6 | APOA4 | PPP1R1 4B | PTK2B |
| IFITM2 | CFD | JCHAIN | IKBKB | DEFB1 | OAS1 | TRIM43 B | TRIM26 |
| IFITM3 | PHB2 | TRIM8 | RNF135 | RELB | PJA2 | TRIM40 | TRIM27 |
| SP100 | IL23R | CLEC7A | SMPD1 | CXCL16 | IL4 | CR2 | TRIM28 |
| CLPB | BCL10 | TRIM5 | DMBT1 | NLRP9 | FES | STYK1 | FYN |
| IFITM1 | WFDC2 | TRIM6 | RNASE8 | NLRP1 | FER | CHUK | NOS2 |
| TIFA | WFDC5 | TRIM4 | H2BS1 | DDX58 | IRF5 | S100B | TRIM21 |
| NOD2 | CFP | RIPK2 | IKBKG | TNK2 | H2BC10 | IRGM | TRIM22 |
| IFNL1 | IFIT1 | TRIM49 C | PGLYRP 2 | CYBB | MFAP4 | FGR | MBL2 |
| IFNL3 | WFDC3 | TRIM49 B | PGLYRP 1 | CYBA | H2BC11 | ZAP70 | |
| IFNL2 | C3 | FCER1G | SETD2 | ISG15 | H2BC12 | LCK | |
| B2M | NUB1 | CAMP | CD74 | MFHAS1 | TRIM58 | ANG | |
| APOL1 | CYP27B 1 | DEFB11 9 | DEFA5 | MYD88 | PYDC1 | TRIM38 | |
| IFNL4 | BPIFA1 | SYK | RARRES 2 | MAVS | TRIM59 | BPI | |
| SNCA | TBK1 | GCH1 | DEFA6 | CLEC6A | IRF7 | PTX3 | |
| S100A1 2 | RPL39 | DEFB11 8 | F12 | LCN2 | SERINC 3 | MASP2 | |
| YES1 | TRIM49 D1 | RNASE7 | DEFA3 | TRIM77 | TRIM56 | TRIM39 | |
| SRMS | PLCG2 | RNASE6 | DEFA4 | FCN2 | SERINC 5 | TRIM32 | |
| APOBEC 3F | SKP2 | DEFB11 4 | ATAD3A | BLK | TRIM64 B | LTF | |

| Innate immune response activating cell surface receptor signaling pathway (GO:0002220) | | | | | | | |
|---|---|---|---|---|---|---|---|
| FBXW11 | KLRD1 | PSMD8 | CREBBP | MUC20 | PRKACB | SYK | RPS6KA 5 |
| TYROBP | PSMC1 | PAK1 | CARD11 | TAB1 | SKP1 | CHUK | PSMB7 |
| MUC3A | RAF1 | PSMD5 | PRKCD | MUC21 | PRKACA | PDPK1 | IKBKB |
| MUC3B | FCN1 | PSMD6 | MUC17 | BCL10 | MUC12 | PSMA6 | PSMB8 |
| PSMB9 | RELA | PSMD3 | KIR2DS 2 | ICAM3 | LYN | PSMA7 | PSMB5 |
| CLEC4C | CLEC10 A | PSMD4 | MUC19 | CUL1 | CARD9 | PSMA4 | PSMB6 |
| PSMC6 | RELB | PSMD1 | NFKB1 | ICAM2 | MUC5AC | PSMA5 | PSMB3 |
| CLEC4D | PSMB11 | PSMD2 | CLEC6A | PRKACG | PSMD11 | PSMA2 | PSMB4 |
| CLEC4E | PSMB10 | MUCL1 | PSME4 | MUC13 | PSMD10 | PSMA3 | PSMB1 |
| PSMC4 | PSMD9 | MAP3K7 | TRAF6 | MUC15 | PSMD13 | PSMA1 | EP300 |
| PSMC5 | MUC1 | MUC4 | PSME2 | PSMF1 | PSMD12 | KRAS | PSMB2 |
| PSMC2 | NRAS | PAK3 | PSME3 | KLRC2 | PSMD14 | SRC | HRAS |
| CD209 | MUC2 | BTRC | TAB3 | FFAR2 | CLEC7A | LILRA2 | FYN |
| PSMC3 | PSMD7 | MUC6 | TAB2 | PLCG2 | FCER1G | PSMA8 | IKBKG |
| CLEC4A | PAK2 | MUC7 | PSME1 | MUC16 | MUC5B | MALT1 | |

| Lymphocyte activation involved in immune response (GO:0002285) | | | | | | | |
|---|---|---|---|---|---|---|---|
| IFNA6 | CD74 | IFNA2 | IFNA17 | CD1C | MDK | F2RL1 | IFNA21 |
| IFNA5 | IFNA8 | IFNA16 | IFNA4 | IFNW1 | LCP1 | IFNB1 | IFNA10 |
| IFNA14 | IFNA7 | IFNA1 | EIF2AK 4 | IFNE | GPR183 | IFNK | CD244 |

| Mucosal immune response (GO:0002385) | | | | | | | |
|---|---|---|---|---|---|---|---|
| DEFA5 | IFNLR1 | RNASE2 | H2BC11 | PLA2G1 B | IFNL2 | FFAR2 | |
| DEFA6 | DEFA1 | BPIFB1 | H2BC12 | PIGR | H2BC21 | NOS2 | |
| DEFA3 | APOA4 | IL4 | RAB17 | OTUD7B | FFAR3 | H2BS1 | |
| DEFA4 | RNASE3 | H2BC10 | LTF | DEFB1 | RPL39 | CAMP | |

| natural killer cell activation involved in immune response (GO:0002323) | | | | | | | |
|---|---|---|---|---|---|---|---|
| IFNA6 | IFNA14 | IFNA7 | IFNA16 | IFNA17 | CORO1A | IFNE | KLRF2 |
| IFNA5 | IFNA8 | IFNA2 | IFNA1 | IFNA4 | IFNW1 | VAMP7 | VAMP2 |
| IFNB1 | IFNK | IFNA21 | IFNA10 | CD244 | | | |

| Negative regulation of immune response (GO:0050777) | | | | | | | |
|---|---|---|---|---|---|---|---|
| SPINK5 | ADCY5 | PRKAR1 A | CALCR | SIRT2 | ADRB3 | GLP2R | PTH |
| CD163 | ADCY4 | RLN3 | LGALS3 | MC3R | CD3G | GPR32 | GPHA2 |
| CD300A | ADCY3 | RLN2 | HTR7 | FCGR2A | NLRC3 | SCTR | FURIN |
| RC3H1 | ADCY2 | HLA-DRB1 | GPR176 | COL3A1 | TREM2 | GIP | PTGER2 |
| MUL1 | ADCY9 | CRHR1 | PRKAR2 B | FCGR2B | ILRUN | PTH2R | TRAF3I P1 |
| ACOD1 | ADCY8 | CALCRL | PCBP2 | IL6 | MC2R | ITCH | GPR45 |
| GCG | ADCY7 | CALCB | PRKAR2 A | FER | FCGR3A | HCK | PTGER4 |
| TAAR1 | ADCY6 | SPI1 | PLCG1 | ADORA2 B | HRH2 | TSHR | NPS |
| FOXP3 | GNG2 | LHB | PLCG2 | ADORA2 A | IRAK3 | ADCYAP 1 | FCGR1A |
| MAPK14 | IFNL1 | CALCA | PRKACB | FSHR | C1QBP | FGR | DHX58 |
| IAPP | CYSLTR 2 | MAPKBP 1 | CGA | MYH9 | DRD1 | PTHLH | MC4R |
| TRAFD1 | CYSLTR 1 | GPR84 | GGT1 | CD247 | HAVCR2 | POMC | ENPP3 |
| ADM2 | IFI16 | RXFP1 | PRKACA | GIPR | LYAR | PPM1B | TRIM27 |
| DPEP1 | GPR150 | SAMSN1 | CCR2 | GPR27 | DRD5 | ADAM17 | FYN |
| PDCD1 | PTGDR | HTR6 | LYN | GPR25 | NPSR1 | MC5R | TRIM21 |
| GPHB5 | YES1 | RXFP2 | GPR15 | GLP1R | RHBDF2 | GHRH | INSL3 |
| VIP | GGT5 | ADCYAP 1R1 | IL10 | ALOX15 | GPR39 | TYRO3 | TSHB |
| SCT | PLK2 | GPR83 | AVPR2 | TNFAIP 3 | CR1 | CRH | |
| RAMP3 | HLA-G | CRHR2 | FSHB | GPR20 | LHCGR | AVP | |
| VIPR2 | CREB1 | HTR4 | AMBP | ADM | P2RY11 | CACTIN | |
| ADCY1 | MC1R | FCRLB | GPBAR1 | ADRB1 | PTGIR | RAMP1 | |
| VIPR1 | GNB1 | GHRHR | NMI | PTH1R | SYK | RAMP2 | |
| PTH2 | PRKAR1 B | PRKACG | LILRB1 | ADRB2 | RNF26 | SRC | |

| Negative regulation of innate immune response (GO:0045824) | | | | | | | |
|---|---|---|---|---|---|---|---|
| CR1 | FAM3A | ISG15 | SERPIN G1 | GRN | DCST1 | IFI16 | A2M |
| PTPN2 | METTL3 | OAS1 | TYRO3 | TNFAIP 3 | NLRC3 | DHX58 | TRIM21 |
| YTHDF2 | NMI | CNOT7 | TTLL12 | NLRC5 | SAMHD1 | SUSD4 | LYAR |
| YTHDF3 | MUL1 | OAS3 | CACTIN | USP18 | RPS19 | SLAMF8 | |
| STAT2 | ACOD1 | TRAFD1 | USP15 | INS | IRAK3 | VSIG4 | |

| neutrophil activation involved in immune response (GO:0002283) | | | | | | | |
|---|---|---|---|---|---|---|---|
| UNC13D | FUCA1 | OLR1 | RAB44 | ITGAX | DERA | ORM1 | C5AR1 |
| ARMC8 | GSDMD | GSN | GLIPR1 | ITGAV | BST2 | GDI2 | COPB1 |
| MCEMP1 | ANXA2 | IST1 | MMP25 | PGM2 | CEACAM 3 | FAF2 | SLC2A3 |
| SCAMP1 | ANXA3 | AGL | CLEC5A | PGM1 | PTPRC | CAB39 | PSEN1 |
| FLG2 | JUP | AOC1 | TXNDC5 | PTPRN2 | RAP2B | AP2A2 | PLAU |
| DYNC1L I1 | FUCA2 | DEFA4 | TOM1 | KRT1 | BST1 | LILRA2 | SRP14 |
| GALNS | NFAM1 | DEFA1 | TMEM17 9B | NBEAL2 | CEACAM 1 | LILRA3 | SLC2A5 |
| TOLLIP | AZU1 | ATP11B | GYG1 | MAPK14 | RAP2C | CST3 | CNN2 |
| PSMC2 | TMC6 | ILF2 | SLC15A 4 | CRISPL D2 | CEACAM 8 | CXCR2 | ADGRG3 |
| PSMC3 | LYZ | ATP11A | ACTR2 | SLPI | PTPRB | CXCR1 | ALDH3B 1 |
| HSPA1A | SIGLEC 5 | TICAM2 | HSP90A A1 | TMEM63 A | CEACAM 6 | STBD1 | SIRPA |
| GLA | SIGLEC 9 | CLEC4C | APAF1 | STXBP2 | ALDOC | RAC1 | ACAA1 |
| NHLRC3 | GPI | CLEC4D | PA2G4 | FGL2 | ACTR10 | ENPP4 | HGSNAT |
| CDK13 | PAFAH1 B2 | RAB31 | NDUFC2 | STXBP3 | ALDOA | CD59 | MMP8 |
| CDA | SERPIN A3 | OLFM4 | TNFRSF 1B | PRCP | RAB7A | CCT8 | HSPA6 |
| HSPA1B | SERPIN A1 | RAB37 | TMBIM1 | CALML5 | RAB5C | SPTAN1 | GGH |
| PRSS2 | FPR2 | S100A7 | DNAJC3 | PNP | SLC44A 2 | CD58 | HSPA8 |
| CYFIP1 | FPR1 | ARL8A | FCGR2A | CPNE1 | HBB | CD55 | CMTM6 |
| PDXK | LRRC7 | ADAM8 | NPC2 | CPNE3 | CTSZ | TBC1D1 0C | MMP9 |
| C3AR1 | IQGAP1 | DSC1 | DNAJC5 | B2M | PTPRJ | LAIR1 | ADA2 |
| FRMPD3 | IQGAP2 | PTPN6 | DSG1 | ARSA | CTSS | CHRNB4 | DNASE1 |
| RETN | TIMP2 | PKP1 | PECAM1 | ARSB | DSN1 | CD300A | MAN2B1 |
| ASAH1 | AP1M1 | S100A9 | VPS35L | XRCC5 | ACTR1B | GCA | MS4A3 |
| CRACR2 A | DYNLL1 | GHDC | SNAP25 | STK11I P | RAP1A | IRAG2 | DOCK2 |
| PSMD7 | SERPIN B3 | S100A8 | SNAP23 | SURF4 | RAP1B | PDAP1 | CHI3L1 |
| PSMD6 | ARG1 | FRK | UBR4 | SLC11A 1 | MAGT1 | HUWE1 | PSMD11 |
| PSMD3 | AHSG | CD177 | FCGR3B | XRCC6 | ORMDL3 | TYROBP | B4GALT 1 |
| ALOX5 | DGAT1 | SLC27A 2 | ALAD | COMMD3 | NEU1 | ATAD3B | PSMD13 |
| PSMD1 | PGAM1 | ROCK1 | PSAP | PRG3 | MLEC | OSCAR | DDX3X |
| STOM | SERPIN B1 | HP | ACP3 | PPBP | QSOX1 | EEF1A1 | PSMD12 |
| PSMD2 | ADAM10 | EPX | CEP290 | RAB27A | CTSH | PPIA | PSMD14 |
| NCSTN | STING1 | APEH | SNAP29 | MIF | CTSG | DOK3 | TNFAIP 6 |
| PRSS3 | LRG1 | NRAS | CR1 | HLA-C | FCER1G | DEGS1 | MOSPD2 |
| S100A1 2 | RNASE3 | PYCARD | DSP | PRG2 | CD36 | CD68 | HMGB1 |
| S100A1 1 | RNASE2 | LAMP2 | RNASET 2 | HLA-B | CTSD | YPEL5 | CXCL1 |
| ELANE | TUBB4B | LAMP1 | CPPED1 | VAT1 | CAMP | PPIE | AGPAT2 |
| PRKCD | SERPIN B6 | RAB24 | PKM | VNN1 | CD33 | FCN1 | PLD1 |
| ATP6AP 2 | ERP44 | KCMF1 | CHIT1 | CSNK2B | CTSC | MPO | HK3 |
| NCKAP1 L | FABP5 | RAB4B | KPNB1 | CYSTM1 | RAB6A | ARHGAP 45 | SIRPB1 |
| HRNR | PSMA5 | TRAPPC 1 | NFASC | MGST1 | CD53 | COTL1 | HEBP2 |
| VAMP7 | PSMA2 | ATP8B4 | PFKL | TRPM2 | CAP1 | CAPN1 | SYNGR1 |
| VAMP8 | SLCO4C 1 | ARPC5 | RAB10 | COMMD9 | CCT2 | OSTF1 | ADGRE3 |
| BIN2 | LAMTOR 1 | KCNAB2 | TSPAN1 4 | LGALS3 | DBNL | IDH1 | DPP7 |
| TCN1 | PLEKHO 2 | EEF2 | VAPA | ANPEP | MME | MGAM | ADGRE5 |
| VAMP2 | LAMTOR 3 | C60RF1 20 | IMPDH1 | FTH1 | SYK | IGF2R | TMEM30 A |
| PADI2 | LAMTOR 2 | NFKB1 | IMPDH2 | CD14 | GAA | ATP6V0 C | PLAUR |
| CFD | SERPIN B12 | ACLY | P2RX1 | GOLGA7 | CREG1 | MANBA | FGR |
| GMFG | GRN | DDOST | RAB14 | ATP6V0 A1 | QPCT | CD93 | GLB1 |
| GNS | PIGR | HPSE | MNDA | CTSB | PGRMC1 | DNAJC1 3 | CAT |
| A1BG | PYGB | LCN2 | BPI | CTSA | ANO 6 | CYBB | S100P |
| ABCA13 | PTAFR | NIT2 | RAB18 | RHOA | SVIP | CYBA | PTX3 |
| CFP | SERPIN B10 | FTL | FOLR3 | PTGES2 | APRT | SDCBP | DYNLT1 |
| C3 | HEXB | RAB5B | ITGB2 | SIGLEC 14 | RAB9B | CKAP4 | LTF |
| PRDX4 | GSTP1 | RAB3D | CANT1 | TUBB | CD47 | DIAPH1 | CLEC12 A |
| CYB5R3 | PYGL | RAB3A | HSP90A B1 | RHOF | DNASE1 L3 | TARM1 | MVP |
| PRDX6 | LPCAT1 | ARHGAP 9 | TCIRG1 | LILRB2 | DNASE1 L1 | SELL | CAND1 |
| PLAC8 | PSMB7 | ATP8A1 | ITGAM | AMPD3 | DYNC1H 1 | PRTN3 | MAPK1 |
| NAPRT | PSMB1 | BRI3 | ITGAL | LILRB3 | CD44 | VCL | GUSB |
| HMOX2 | METTL7 A | HVCN1 | FCAR | RHOG | ORM2 | CRISP3 | |
| LTA4H | AGA | GPR84 | TTR | NME2 | CSTB | ATP6V1 D | |
| ATG7 | PGLYRP 1 | STK10 | GM2A | TLR2 | CD63 | VCP | |

| Positive regulation of cytokine production involved in immune response (GO:0002720) | | | | | | | |
|---|---|---|---|---|---|---|---|
| FZD5 | WNT5A | TRAF2 | SLC7A5 | TNFSF4 | NOD2 | CLEC7A | PLCG2 |
| XCL1 | LAPTM5 | HLA-G | RAET1G | TRAF6 | KIR2DL 4 | TRIM6 | B2M |
| CD160 | HLA-A | HLA-E | KIT | F2RL1 | MALT1 | FFAR3 | MAP3K7 |
| SPHK2 | SCIMP | HLA-F | LACC1 | CD226 | INAVA | FFAR2 | TNFRSF 14 |
| CD55 | | | | | | | |

| Positive regulation of immune effector process (GO:0002699) | | | | | | | |
|---|---|---|---|---|---|---|---|
| CCR2 | IL2 | IL23A | CD46 | CD81 | DDX60 | DHX58 | LGALS9 |
| IL10 | USP17L 2 | TNFSF4 | HLA-DRB1 | PHB | HLA-DMB | LBP | PCK1 |
| XBP1 | IL1B | IFNG | ANKRD1 7 | PLA2G5 | MYB | MYO18A | IL12RB 1 |
| LILRB1 | PTPRC | HLA-DRA | IL23R | PTPRJ | RPS19 | IL12B | APPL2 |

| positive regulation of immune response (GO:0050778) | | | | | | | |
|---|---|---|---|---|---|---|---|
| AKIRIN 2 | EIF2AK 4 | POLR3C | IFNK | FPR2 | STX4 | NLRP10 | PLA2G1 B |
| CD74 | NMI | IL23A | ADAM8 | NOD2 | PCK1 | F2RL1 | IL23R |
| SIGLEC 16 | LILRB1 | POLR3D | IL6ST | IFNL1 | IL12RB 1 | CD226 | IFI35 |
| TASL | SIRT1 | TNFSF4 | SKAP1 | PYCARD | GBP5 | ZP4 | ITGAM |
| TGFB2 | EREG | POLR3F | HRG | MYB | SASH3 | CD46 | CD1D |
| IL10 | IL2 | IFNG | CD177 | GPR151 | XBP1 | ZP3 | HLA-DMB |
| IL15 | PTPRC | POLR3G | DHX9 | NLRP3 | SH2D1B | HLA-DRB1 | CCL5 |
| SPHK2 | FCGR2B | HLA-DRA | CD81 | FADD | CYBA | ITGB2 | IFNB1 |
| RSAD2 | POLR3B | TLR8 | KARS1 | CCR7 | PLSCR1 | GRN | IL12B |
| RIOK3 | SLC15A 4 | LGALS9 | | | | | |

| Positive regulation of production of molecular mediator of immune response (GO:0002702) | | | | | | | |
|---|---|---|---|---|---|---|---|
| IL10 | LAPTM5 | PTPRC | CLCF1 | CD244 | PHB | FFAR3 | EPHB2 |
| XBP1 | SCIMP | IL6 | F2RL1 | MZB1 | PTPN22 | DNAJB9 | TNFRSF 14 |
| XCL1 | HLA-E | RBP4 | TLR9 | CD86 | NOD2 | FFAR2 | SASH3 |
| SPHK2 | IL2 | LACC1 | VAMP3 | PHB2 | INAVA | STX4 | |
| WNT5A | SLC7A5 | TNFSF4 | GPI | IL21 | TRIM6 | TNFRSF 4 | |

| regulation of adaptive immune response (GO:0002819) | | | | | | | |
|---|---|---|---|---|---|---|---|
| RIPK3 | AHR | ADCY7 | IRF1 | FADD | IL6ST | | |
| ALOX15 | SIRT1 | IL4I1 | FCGR2B | TRIM27 | SKAP1 | | |
| EIF2AK 4 | SAMSN1 | PYCARD | TNFSF4 | IRF7 | SASH3 | | |

| Regulation of cytokine production involved in immune response (GO:0002718) | | | | | | | |
|---|---|---|---|---|---|---|---|
| CCR2 | WNT5A | LAPTM5 | TNF | INAVA | APOA1 | CLC | FFAR2 |
| IL10 | TRIL | APOA2 | SCIMP | SLC7A5 | LACC1 | BTK | TNFRSF 14 |
| SPHK2 | LILRB1 | NOD2 | TNFRSF 1B | TRIM6 | F2RL1 | FFAR3 | TRPM4 |

| regulation of humoral immune response (GO:0002920) | | | | | | | |
|---|---|---|---|---|---|---|---|
| CFHR1 | CD81 | C8B | CR1 | CFB | CFI | VTN | C7 |
| FCGR2B | C3AR1 | C4A | CR2 | C1QC | C5AR1 | C2 | C9 |
| CXCL13 | PROS1 | C8G | F2 | C1QB | CFH | CPN2 | CD19 |
| CFHR2 | C4BPA | C4B | SERPIN G1 | C1QA | C1S | C3 | CD59 |
| CR1L | C8A | C1QBP | ZP4 | PHB2 | C5AR2 | CPN1 | CD55 |
| CFHR5 | CLU | SUSD4 | CD46 | C1R | GATA6 | C5 | |
| CFHR4 | C4BPB | CCR7 | ZP3 | CPB2 | CFP | C6 | |

| Regulation of immune effector process (GO:0002697) | | | | | | | |
|---|---|---|---|---|---|---|---|
| ZNF683 | CFHR4 | CLU | SUSD4 | HLA-DRB1 | CFI | C2 | C9 |
| CFHR1 | HLA-DRA | C4BPB | CR1 | C1QC | C5AR1 | CPN2 | CD19 |
| FCGR2B | CD81 | C8B | CR2 | C1QB | CFH | C3 | CD59 |
| IRF4 | C3AR1 | C4A | F2 | C1QA | C1S | CPN1 | CD55 |
| CFHR2 | PROS1 | C8G | SERPIN G1 | PHB2 | C5AR2 | C5 | |
| CR1L | C4BPA | C4B | CD46 | C1R | CFP | C6 | |
| CFHR5 | C8A | C1QBP | CFB | CPB2 | VTN | C7 | |

| Regulation of immune response (GO:0050776) | | | | | | | |
|---|---|---|---|---|---|---|---|
| SPINK5 | CLEC4G | HLA-C | COL17A 1 | ITGA4 | SIGLEC 9 | CD300L D | KLRF1 |
| TGFB2 | CD200 | TIRAP | C17ORF 99 | RIPK3 | FCGR2C | MADCAM 1 | NECTIN 2 |
| LAG3 | IFITM1 | TRAF3 | SPPL2B | SFTPD | SIGLEC 7 | FCER1A | ITGB2 |
| CD300A | FGL1 | HLA-A | SPPL2A | AMBP | CD22 | CD300L F | ITGB1 |
| CD160 | CD81 | HLA-B | ICAM3 | PILRA | CD40 | CD300L G | COLEC1 2 |
| CRTAM | PVR | HLA-G | ICAM4 | LILRB1 | DENND1 B | CD34 | PLA2G1 B |
| RSAD2 | AHR | HLA-E | ICAM5 | LILRB2 | PTPN22 | CD33 | LILRA1 |
| TYROBP | ADCY7 | HLA-F | CD1D | PILRB | KIR2DL 1 | CXADR | ITGAL |
| VCAM1 | LRP8 | TREML2 | CD1C | CD200R 1 | CD3G | PIANP | SAMHD1 |
| FOXP3 | IFNL1 | RAET1E | ICAM1 | NCR2 | KIR2DL 2 | SYK | RNF135 |
| COL1A2 | HERC5 | COL2A1 | CD1B | IL4 | TREM1 | ERAP1 | FCGR1A |
| FOXP1 | GPR151 | TREML1 | SLA2 | IRF1 | KIR2DL 3 | ELMOD2 | DHX58 |
| OSCAR | SLAMF7 | PLSCR1 | ICAM2 | NCR3 | CD3E | KIR3DL 1 | CSK |
| COL1A1 | SLAMF 6 | CD8B | C3 | FCGR2A | TREM2 | NCR3LG 1 | ITGB7 |
| CXCL13 | B2M | CD8A | CD1A | COL3A1 | KIR2DL 4 | KIR3DL 2 | APPL1 |
| TNFSF4 | MICA | TREML4 | FCRLB | FCGR2B | ZMPSTE 24 | FGR | LAIR2 |
| JAML | MICB | SELL | IL4I1 | IRF4 | CD3D | FKBP1A | LAIR1 |
| CD300E | N4BP1 | GPR108 | KLRK1 | BCL6 | SPN | CLEC2B | APPL2 |
| KLRD1 | CD96 | KLRB1 | CD19 | NCR1 | FCGR3A | XIAP | |
| CD300C | KIR2DS 1 | BIRC2 | KLRC1 | HLA-DRA | CASP8 | CD226 | |
| ULBP1 | KIR2DS 2 | BIRC3 | CD99 | HCST | FCGR3B | CLEC2D | |
| PDCD1 | SH2D1A | HLA-DRB1 | LYN | IRF7 | TKFC | CD40LG | |
| ULBP3 | SH2D1B | CD276 | IL15 | CD247 | CD300L B | NPDC1 | |

| Regulation of innate immune response (GO:0045088) | | | | | | | |
|---|---|---|---|---|---|---|---|
| PTPN1 | IRF1 | TLR8 | NLRC3 | LYAR | PLSCR1 | ABCE1 | DHX58 |
| AKIRIN 2 | FCGR2B | IFNK | TREM2 | N4BP1 | CDC37 | RNASEL | CCL5 |
| TASL | POLR3B | ADAM8 | LRP8 | ERAP1 | XIAP | HSP90A B1 | IFNB1 |
| NMI | POLR3C | PTPN6 | CASP8 | SH2D1B | TYRO3 | IFI35 | RIOK3 |
| MUL1 | POLR3D | DHX9 | TKFC | TIRAP | CACTIN | USP18 | SLC15A 4 |
| PTPN11 | TRAFD1 | TNFAIP 3 | IRAK3 | HLA-E | BIRC2 | CD1D | TRIM21 |
| ACOD1 | POLR3F | FPR2 | IFI16 | PARP9 | BIRC3 | SAMHD1 | APPL1 |
| EREG | POLR3G | PTPN22 | GBP5 | FGR | IFNAR2 | RNF135 | APPL2 |

| Antigen processing and presentation | | | | | | | |
|---|---|---|---|---|---|---|---|
| CTSB | TAP2 | KLRD1 | B2M | NFYB | KIR3DL 2 | CD8A | HSP90A B1 |
| CD74 | HSPA4 | CALR | HLA-DOB | KIR2DS 2 | HLA-A | CANX | IFI30 |
| HSP90A A1 | TAP1 | HSPA1B | KIR2DL 5A | NFYC | HLA-B | LGMN | HLA-DMB |
| KLRC3 | TAPBP | TNF | CIITA | KIR2DS 3 | KIR3DL 3 | PSME2 | HLA-DPA1 |
| HSPA6 | CD4 | KIR2DL 1 | HLA-DQA2 | KIR2DS 4 | HLA-G | PSME3 | KLRC1 |
| HSPA5 | RFX5 | KIR2DL 2 | HLA-DOA | KIR2DS 5 | HLA-E | HLA-DPB1 | KLRC2 |
| HSPA1L | IFNG | KIR2DL 3 | HLA-DQA1 | HLA-C | HLA-F | PSME1 | NFYA |
| KLRC4 | HSPA1A | KIR2DL 4 | PDIA3 | RFXANK | CREB1 | HLA-DRB4 | HLA-DMA |
| HSPA8 | HLA-DRA | CTSS | HLA-DRB5 | KIR3DL 1 | CD8B | HLA-DRB3 | |
| HSPA2 | HLA-DQB1 | CTSL | KIR2DS 1 | CD8B2 | RFXAP | HLA-DRB1 | |

| B cell receptor signaling pathway | | | | | | | |
|---|---|---|---|---|---|---|---|
| LYN | LILRB4 | SOS2 | PIK3R3 | BLNK | PIK3R1 | LILRA3 | PPP3R2 |
| JUN | VAV1 | PTPN6 | INPPL1 | CARD11 | BTK | LILRA4 | CD79A |
| CD72 | LILRB5 | NFKBIE | PIK3R2 | MAP2K2 | KRAS | DAPP1 | CD19 |
| IGH | VAV2 | RAF1 | PPP3CA | CR2 | GRB2 | LILRA5 | MAPK3 |
| NFATC3 | VAV3 | CD22 | NRAS | SYK | PIK3AP 1 | LILRA6 | RAC3 |
| LILRB1 | NFKBIB | SOS1 | PPP3CB | MAP2K1 | BCL10 | MALT1 | RAC1 |
| LILRB2 | NFKBIA | RELA | PPP3CC | CHUK | PIK3CD | INPP5D | MAPK1 |
| NFATC2 | FCGR2B | GSK3B | AKT3 | FOS | LILRA1 | IKBKB | HRAS |
| NFATC1 | PIK3CB | IFITM1 | AKT1 | PRKCB | LILRA2 | PPP3R1 | RAC2 |
| LILRB3 | PIK3CA | CD81 | AKT2 | NFKB1 | RASGRP 3 | CD79B | PLCG2 |
| IKBKG | | | | | | | |

| Chemokine signaling pathway | | | | | | | |
|---|---|---|---|---|---|---|---|
| BAD | GNG2 | PIK3R1 | CCR1 | CCL27 | GRK1 | PLCB4 | CXCR1 |
| CXCL10 | CRKL | GNB3 | CCL24 | CXCL8 | RAP1A | CRK | ARRB1 |
| CXCL11 | NRAS | GNB2 | CCL23 | SHC3 | GRK4 | KRAS | CCL7 |
| CXCL12 | GNG4 | GNAQ | CCL22 | GSK3B | RAP1B | PLCB2 | CXCR4 |
| CXCL13 | PAK1 | GNB5 | CCL21 | SHC4 | GRK3 | PLCB3 | CXCR3 |
| ELMO1 | GNG3 | GNB4 | CCL20 | CXCL9 | GRK6 | PLCB1 | CCL5 |
| NCF1 | GNG5 | GRB2 | XCL1 | GSK3A | GRK5 | CCL15 | MAPK3 |
| PF4 | AKT3 | PXN | BRAF | CCL3L1 | GRK7 | CCL14 | CCL4 |
| RAF1 | PREX1 | CCL4L1 | GNG13 | SHC1 | CCR9 | SRC | RAC3 |
| RELA | GNG8 | CCL4L2 | GNG12 | SHC2 | CCR8 | CCL13 | PTK2B |
| ROCK1 | GNG7 | RASGRP 2 | VAV1 | CCL3L3 | CCR7 | CCL11 | CCL3 |
| ADCY1 | AKT1 | PRKACG | VAV2 | PIK3R5 | CCR6 | CX3CR1 | RAC1 |
| ROCK2 | AKT2 | GNG11 | VAV3 | PIK3R3 | CCR5 | CXCR6 | CCL2 |
| ADCY5 | XCR1 | GNG10 | NFKBIB | CXCL2 | CCR4 | CXCR5 | MAPK1 |
| ADCY4 | STAT5B | PLCG1 | NFKBIA | CXCL1 | CCR3 | WAS | HRAS |
| ADCY3 | PRKCD | PLCG2 | PIK3CB | PIK3R2 | PF4V1 | PIK3CD | RAC2 |
| ADCY2 | MAP2K1 | PRKACB | PIK3CA | CXCL3 | STAT1 | ARRB2 | CCL1 |
| CXCL14 | PPBP | PRKACA | XCL2 | CX3CL1 | STAT2 | GNAI1 | CCL19 |
| ADCY9 | PARD3 | CCR10 | SOS2 | CXCL6 | CHUK | GNAI2 | JAK3 |
| FOXO3 | PRKCB | CCL26 | DOCK2 | CXCL5 | STAT3 | GNAI3 | IKBKG |
| ADCY8 | NFKB1 | CCR2 | ITK | PIK3R6 | HCK | PIK3CG | CCL18 |
| ADCY7 | PTK2 | LYN | CCL28 | GNGT2 | TIAM1 | IKBKB | CCL17 |
| CXCL16 | PRKCZ | CCL25 | BCAR1 | GRK2 | CDC42 | CXCR2 | JAK2 |
| ADCY6 | GNB1 | RHOA | SOS1 | GNGT1 | FGR | CCL8 | CCL16 |

| Complement and coagulation cascades | | | | | | | |
|---|---|---|---|---|---|---|---|
| F11 | SERPIN A1 | SERPIN A5 | CR2 | F8 | ITGB2 | F13A1 | C7 |
| F10 | MASP1 | C4A | FGG | F9 | C1QB | PLAU | C9 |
| F12 | SERPIN E1 | KNG1 | F2R | PROCR | CFD | PLAT | ITGAX |
| SERPIN D1 | C3AR1 | C8G | VWF | SERPIN G1 | C1QA | PLG | VSIG4 |
| CFHR1 | PROS1 | C4B | SERPIN F2 | F13B | C1R | ITGAM | A2M |
| CFHR3 | C4BPA | BDKRB1 | F2 | F2RL2 | CPB2 | VTN | CD59 |
| CFHR2 | C8A | BDKRB2 | PLAUR | MASP2 | SERPIN C1 | C2 | MBL2 |
| CR1L | CLU | FGA | F3 | CD46 | CFI | C3 | CD55 |
| CFHR5 | C4BPB | CR1 | F5 | F2RL3 | C5AR1 | C5 | |
| CFHR4 | TFPI | FGB | F7 | CFB | CFH | THBD | |
| KLKB1 | C8B | SERPIN B2 | PROC | C1QC | C1S | C6 | |

| Cytosolic DNA-sensing pathway | | | | | | | |
|---|---|---|---|---|---|---|---|
| ZBP1 | POLR3A | POLR3G | CASP1 | IFNA17 | IFNA6 | IFNA4 | IFNB1 |
| POLR3G L | IL6 | POLR3H | RIPK1 | STING1 | IFNA5 | IKBKB | POLR2H |
| RIPK3 | POLR3B | IRF7 | IL33 | NFKB1 | IFNA8 | TBK1 | IKBKG |
| IL18 | POLR3C | POLR3K | IFNA13 | AIM2 | IFNA7 | POLR2E | POLR2K |
| CXCL10 | POLR3D | RELA | IFNA14 | MAVS | IFNA2 | CCL5 | IKBKE |
| NFKBIB | POLR3E | CGAS | DDX58 | POLR1C | CCL4L1 | CCL4 | POLR2L |
| IL1B | POLR3F | ADAR | IFNA16 | POLR1D | IFNA1 | POLR2F | IFNA21 |
| NFKBIA | IRF3 | PYCARD | CHUK | IFNA10 | CCL4L2 | TREX1 | |

| Fc epsilon RI signaling pathway | | | | | | | |
|---|---|---|---|---|---|---|---|
| LYN | MAPK14 | SOS2 | NRAS | MAP2K3 | ALOX5A P | INPP5D | RAC2 |
| IL13 | MAPK11 | MS4A2 | AKT3 | PLA2G4 E | PLA2G4 B | MAPK9 | PLCG2 |
| GAB2 | IL5 | RAF1 | ALOX5 | SYK | PRKCA | MAPK8 | FYN |
| IGH | IL4 | LAT | AKT1 | PLA2G4 F | PIK3R1 | MAPK3 | MAP2K6 |
| VAV1 | MAPK12 | SOS1 | AKT2 | MAP2K1 | JMJD7-PLA2G4 B | RAC3 | MAP2K7 |
| VAV2 | MAPK10 | CSF2 | FCER1A | PDPK1 | BTK | PLCG1 | |
| IL3 | PIK3CB | PIK3R3 | FCER1G | PLA2G4 C | KRAS | RAC1 | |
| MAPK13 | PIK3CA | TNF | MAP2K4 | PLA2G4 D | GRB2 | MAPK1 | |
| VAV3 | LCP2 | PIK3R2 | MAP2K2 | PLA2G4 A | PIK3CD | HRAS | |

| Fc gamma R-mediated phagocytosis | | | | | | | |
|---|---|---|---|---|---|---|---|
| GSN | CFL2 | PLA2G4 B | PLPP1 | LIMK1 | PIK3CA | WASF2 | PIK3CD |
| IGH | AKT1 | PRKCB | PLA2G6 | SPHK2 | DOCK1 | WASF3 | FCGR1A |
| MARCKS | CFL1 | ARPC5 | ACTR3B | LIMK2 | LAT | MARCKS L1 | MAPK3 |
| NCF1 | AKT2 | PRKCA | ACTR3C | SPHK1 | ARPC1A | SYK | RAC1 |
| RAF1 | PRKCG | VASP | INPP5D | GAB2 | ARPC1B | MYO10 | MAPK1 |
| ARPC5L | PRKCD | ARPC3 | PIP5K1 A | VAV1 | PIK3R3 | HCK | RAC2 |
| ASAP2 | PLA2G4 E | PIK3R1 | PIP5K1 B | VAV2 | PIK3R2 | CDC42 | |
| INPPL1 | PLA2G4 F | ARPC4 | PLCG1 | DNM2 | PLD2 | RPS6KB 2 | |
| ASAP3 | MAP2K1 | BIN1 | PIP5K1 C | VAV3 | PLD1 | RPS6KB 1 | |
| CRKL | PLA2G4 C | ARPC2 | PLCG2 | PTPRC | FCGR3A | JMJD7-PLA2G4 B | |
| PAK1 | PLA2G4 D | AMPH | ACTR3 | FCGR2A | SCIN | CRK | |
| ASAP1 | PRKCE | PLPP3 | LYN | FCGR2B | FCGR3B | ARF6 | |
| AKT3 | PLA2G4 A | PLPP2 | ACTR2 | PIK3CB | WASF1 | WAS | |

| Hematopoietic cell lineage | | | | | | | |
|---|---|---|---|---|---|---|---|
| IL4R | CSF2 | IL7R | CD14 | IL5 | FCER2 | HLA-DQA1 | ITGB3 |
| IL1R1 | CSF1 | HLA-DRB1 | IL6R | IL1B | CD22 | CR1 | ITGAM |
| IL1R2 | ITGA2B | CSF1R | IL11 | IL4 | GYPA | CR2 | HLA-DMB |
| IGH | TNF | TFRC | ITGA1 | IL7 | CD3G | MME | FCGR1A |
| CD2 | FLT3LG | IL5RA | ITGA5 | IL6 | CD3E | IL11RA | HLA-DPA1 |
| CD5 | HLA-DOB | CD1E | ITGA4 | KITLG | CD3D | GP1BB | CD59 |
| CD4 | HLA-DOA | CSF2RA | ITGA3 | IL3RA | THPO | GP1BA | HLA-DMA |
| CD7 | HLA-DRB5 | CD1D | ITGA2 | HLA-DRA | CD38 | EPOR | CD55 |
| CR1L | CD8B2 | CD1C | DNTT | HLA-DQB1 | CD37 | KIT | |
| CD9 | CD8B | CD1B | GP5 | IL9R | CD36 | IL2RA | |
| CSF3R | CD8A | CD1A | GP9 | ITGA6 | CD34 | HLA-DPB1 | |
| EPO | HLA-DRB4 | ANPEP | IL3 | CD24 | HLA-DQA2 | CD44 | |
| CSF3 | HLA-DRB3 | CD19 | IL1A | MS4A1 | CD33 | FLT3 | |

| IL-17 signaling pathway | | | | | | | |
|---|---|---|---|---|---|---|---|
| USP25 | MAPK11 | DEFB4B | CXCL2 | IL17RA | FOSL1 | IL17B | CCL7 |
| JUN | CXCL10 | S100A9 | TNF | JUND | TRAF5 | CEBPB | MAPK3 |
| HSP90A A1 | MMP13 | S100A8 | CXCL1 | MUC5B | TRAF4 | CCL11 | CCL2 |
| MMP1 | IL5 | DEFB4A | ELAVL1 | CHUK | FOSB | HSP90A B1 | MAPK1 |
| CCL20 | IL1B | CXCL8 | CXCL3 | FOS | TRAF6 | TRAF3I P2 | IKBKG |
| MMP3 | IL4 | RELA | HSP90B 1 | TRAF3 | LCN2 | IL25 | MAPK7 |
| IL13 | NFKBIA | GSK3B | CXCL6 | TRAF2 | ANAPC5 | PTGS2 | MAPK6 |
| MUC5AC | MAPK12 | CSF3 | CXCL5 | IL17RE | TAB3 | S100A7 A | IKBKE |
| MMP9 | MAPK10 | CSF2 | CASP8 | NFKB1 | IL17F | IKBKB | CCL17 |
| MAPK15 | IL6 | SRSF1 | CASP3 | IL17RC | TAB2 | MAPK9 | MAPK4 |
| MAPK13 | IFNG | TRADD | FADD | IL17RB | IL17D | MAPK8 | |
| MAPK14 | S100A7 | TNFAIP 3 | MAP3K7 | IL17A | IL17C | TBK1 | |

| Intestinal immune network for IgA production | | | | | | | |
|---|---|---|---|---|---|---|---|
| TGFB1 | IL15 | IL4 | CD28 | CCL28 | PIGR | HLA-DPA1 | HLA-DQA2 |
| AICDA | ITGA4 | CXCL12 | HLA-DPB1 | HLA-DRB1 | CD80 | CCR9 | ICOS |
| CCL25 | TNFSF1 3 | IL6 | CD40LG | CD86 | TNFSF1 3B | MADCAM 1 | CCR10 |
| IL15RA | IGH | LTBR | MAP3K1 4 | CD40 | HLA-DMB | ITGB7 | HLA-DOA |
| IL10 | IL2 | HLA-DRA | HLA-DRB4 | TNFRSF 13C | CXCR4 | HLA-DOB | ICOSLG |
| HLA-DRB5 | IL5 | HLA-DQB1 | HLA-DRB3 | TNFRSF 13B | TNFRSF 17 | HLA-DMA | HLA-DQA1 |

| Leukocyte transendothelial migration | | | | | | | |
|---|---|---|---|---|---|---|---|
| ACTN4 | ROCK2 | PRKCA | F11R | VAV2 | RAPGEF 4 | CLDN9 | CXCR4 |
| ACTN1 | CLDN3 | VASP | ICAM1 | VAV3 | ITK | CLDN8 | CLDN25 |
| VCAM1 | CLDN2 | PTK2 | ARHGAP 5 | AFDN | BCAR1 | SIPA1 | CLDN24 |
| MAPK13 | CLDN1 | OCLN | RASSF5 | CLDN11 | PIK3R3 | ITGB2 | CTNNA2 |
| MAPK14 | MYL12B | MYL12A | PLCG1 | CLDN10 | PIK3R2 | ITGB1 | PTK2B |
| MAPK11 | ARHGAP 35 | MYLPF | PLCG2 | PIK3CB | CD99L2 | CTNNB1 | CTNNAL |
| MYL5 | ACTB | PIK3R1 | CD99 | CLDN16 | THY1 | PIK3CD | RAC1 |
| MAPK12 | CDH5 | ESAM | RHOA | PIK3CA | RAP1A | ITGAM | RAC2 |
| CXCL12 | MYL10 | EZR | ITGA4 | CLDN15 | RAP1B | GNAI1 | CTNNA3 |
| MYL7 | JAM2 | VCL | MMP2 | CLDN14 | CDC42 | ITGAL | |
| MYL2 | JAM3 | NCF2 | MSN | CLDN19 | CLDN7 | GNAI2 | |
| NCF1 | PRKCG | PXN | RHOH | PECAM1 | CLDN34 | GNAI3 | |
| MYL9 | PRKCB | NCF4 | PTPN11 | RAPGEF 3 | CLDN6 | CLDN23 | |
| ROCK1 | CYBB | TXK | MMP9 | CLDN18 | CLDN5 | CLDN22 | |
| CTNND1 | CYBA | ACTG1 | VAV1 | CLDN17 | CLDN4 | CLDN20 | |

| NOD-like receptor signaling pathway | | | | | | | |
|---|---|---|---|---|---|---|---|
| GBP7 | ANTXR2 | CASR | BIRC3 | CARD8 | CXCL8 | CAMP | IFNA8 |
| DEFA5 | TANK | PRKCD | IFNA10 | IRAK4 | YWHAE | STAT1 | RNASEL |
| DEFA6 | RNF31 | IFNA16 | IFNAR2 | IL18 | MEFV | STAT2 | IFNA7 |
| DEFA3 | PYCARD | IFNA17 | TXN2 | CARD6 | ITPR2 | CHUK | IFNA2 |
| DEFA4 | ANTXR1 | NEK7 | DEFB10 3B | OAS1 | TNFAIP 3 | ERBIN | HSP90A B1 |
| DEFA1 | DHX33 | CYBB | GABARA P | TYK2 | ITPR3 | STING1 | IFNA1 |
| TRPV2 | NLRP7 | TRAF3 | DEFB10 3A | NFKBIB | CXCL2 | P2RX7 | IFNA4 |
| TICAM1 | IFI16 | CYBA | TXN | IL1B | NLRC4 | AIM2 | IKBKB |
| MAPK13 | NLRP6 | TRAF2 | BRCC3 | NFKBIA | CXCL1 | PLCB4 | GPRC6A |
| MAPK14 | MFN2 | NFKB1 | TRPM2 | IL6 | CXCL3 | NLRP12 | MAPK9 |
| ATG12 | NLRP3 | MYD88 | TBK1 | OAS2 | ITPR1 | XIAP | MAPK8 |
| MAPK11 | GBP1 | MAVS | TP53BP 1 | OAS3 | CASP8 | TXNIP | NAMPT |
| MAPK12 | NLRP1 | TRAF5 | TRPM7 | IRF3 | SHARPI N | PKN1 | CCL5 |
| MAPK10 | GBP3 | TRAF6 | ATG5 | PYDC1 | PSTPIP 1 | SUGT1 | MAPK3 |
| ATG16L 1 | MAP3K7 | BCL2L1 | IFNA21 | BCL2 | PANX1 | VDAC3 | IFNB1 |
| TRIP6 | GBP2 | CARD18 | DNM1L | IRF9 | CASP12 | VDAC2 | CCL2 |
| RBCK1 | GBP5 | TAB3 | CTSB | PYDC2 | CASP4 | PKN2 | MAPK1 |
| NAIP | MFN1 | CARD16 | RHOA | IRF7 | CASP5 | VDAC1 | IKBKG |
| RELA | GBP4 | TAB2 | GSDMD | DEFB4B | FADD | PLCB2 | IKBKE |
| NLRX1 | IFNA13 | CARD17 | JUN | PYDC5 | RIPK2 | PLCB3 | JAK1 |
| NOD1 | IFNA14 | TAB1 | HSP90A A1 | TLR4 | DEFA1B | PLCB1 | |
| NOD2 | GABARA PL2 | BIRC2 | RIPK3 | DEFB4A | CASP1 | IFNA6 | |
| TNF | GABARA PL1 | IFNAR1 | CARD9 | MCU | RIPK1 | IFNA5 | |

| Natural killer cell mediated cytotoxicity | | | | | | | |
|---|---|---|---|---|---|---|---|
| KLRC3 | MICA | PRKCB | FASLG | VAV2 | SHC1 | IFNGR2 | PIK3CD |
| IGH | MICB | TNFRSF 10B | ICAM1 | VAV3 | SHC2 | GZMB | ITGAL |
| TYROBP | IFNA13 | HLA-A | ICAM2 | NCR2 | PIK3R3 | KIR3DL 1 | MAPK3 |
| FAS | PRKCG | TNFRSF 10A | PPP3R1 | NCR3 | KIR2DL 1 | KIR3DL 2 | RAC3 |
| KLRD1 | IFNA14 | PRKCA | PPP3R2 | PIK3CB | PIK3R2 | KIR3DL 3 | PTK2B |
| ULBP1 | MAP2K2 | HLA-B | KLRK1 | PIK3CA | KIR2DL 2 | ZAP70 | IFNB1 |
| ULBP3 | KIR2DS 1 | HLA-G | PLCG1 | NCR1 | KIR2DL 3 | LCK | RAC1 |
| PTPN6 | IFNA16 | HLA-E | KLRC1 | IFNG | PPP3CA | CD48 | MAPK1 |
| ULBP2 | KIR2DS 2 | RAET1E | TNFSF1 0 | HCST | KLRC4-KLRK1 | KRAS | HRAS |
| RAF1 | MAP2K1 | RAET1G | KLRC2 | LCP2 | PPP3CB | IFNA6 | RAC2 |
| CSF2 | KIR2DS 3 | PIK3R1 | PLCG2 | CD247 | FCGR3A | ITGB2 | FYN |
| TNF | IFNA17 | RAET1L | IFNA21 | SOS2 | PPP3CC | IFNA5 | BID |
| ARAF | SH2D1A | GRB2 | PTPN11 | CD244 | FCGR3B | IFNA8 | |
| NRAS | KIR2DS 4 | IFNAR1 | NFATC2 | LAT | CASP3 | IFNA7 | |
| PAK1 | SH2D1B | IFNA10 | NFATC1 | SOS1 | FCER1G | IFNA2 | |
| SH3BP2 | KIR2DS 5 | IFNAR2 | BRAF | SHC3 | IFNGR1 | IFNA1 | |
| KIR2DL 5A | HLA-C | PRF1 | VAV1 | SHC4 | SYK | IFNA4 | |

| Neutrophil extracellular trap formation | | | | | | | |
|---|---|---|---|---|---|---|---|
| H2AZ2 | H2AB2 | H2AC1 | H2AC18 | H2BC18 | PIK3R2 | H4-16 | PIK3CD |
| H2BC8 | H2AB3 | CYBA | C3 | TLR2 | FPR3 | MTOR | ITGAM |
| H2AZ1 | MPO | PRKCA | H2AC19 | H2BC13 | FCGR3A | H3-4 | HDAC9 |
| H2BC9 | H2AB1 | NFKB1 | H2AC12 | H2BC14 | FCGR3B | PLCB4 | CLCN4 |
| H2BC6 | MACROH 2A2 | H2BE1 | C5 | FCGR2A | CLEC7A | H3-5 | ITGAL |
| H2BC7 | MACROH 2A1 | SELP | H2AC13 | H2BC15 | CASP4 | VDAC3 | CLCN3 |
| H2BC4 | ACTB | PIK3R1 | H2AC14 | CLCN5 | H3C2 | VDAC2 | HDAC7 |
| H2BC5 | AKT3 | H2AC20 | H2AC15 | H2AJ | H3C1 | VDAC1 | HDAC8 |
| IGH | AKT1 | H4C15 | H2AC11 | PIK3CB | H3C4 | PLCB2 | FCGR1A |
| H2BC3 | AKT2 | H4C14 | H4C1 | PIK3CA | CASP1 | SLC25A 6 | H3C14 |
| H2BC1 | MAP3K7 | H2AC21 | PLCG1 | H2BC10 | CTSG | SLC25A 5 | H2BC21 |
| MAPK13 | H2BU1 | H4C13 | H4C3 | H2BC11 | H3C3 | PLCB3 | H3C15 |
| MAPK14 | ELANE | PADI4 | PLCG2 | H2BC12 | H3C6 | PLCB1 | H3C12 |
| MAPK11 | H4C9 | H4C12 | H4C2 | TLR8 | FGA | ITGB2 | H3C13 |
| MAPK12 | PRKCG | H4C11 | ATG7 | TLR7 | H3-3B | HDAC5 | MAPK3 |
| HAT1 | H4C8 | NCF2 | H4C5 | SIGLEC 9 | H3C8 | HDAC6 | H3C10 |
| CR1L | MAP2K2 | HDAC11 | H2BW2 | TLR4 | CAMP | H2AX | H3C11 |
| SLC25A 31 | H2AC7 | C5AR1 | H4C4 | H2AW | H3C7 | SELPLG | RAC1 |
| PPIF | MAP2K1 | HDAC10 | H4C7 | H3-3A | CR1 | SRC | MAPK1 |
| NCF1 | H2AC8 | NCF4 | H4C6 | SLC25A 4 | FGB | HDAC3 | RAC2 |
| RAF1 | H2AC6 | ACTG1 | H2BW1 | FPR2 | FGG | HDAC4 | H2BS1 |
| RELA | H2AC4 | AGER | GSDMD | FPR1 | SYK | HDAC1 | |
| AQP9 | PRKCB | H2AC16 | AZU1 | PIK3R3 | VWF | HDAC2 | |
| ITGA2B | CYBB | H2AC17 | H2BC17 | HMGB1 | GP1BA | ITGB3 | |

| Platelet activation | | | | | | | |
|---|---|---|---|---|---|---|---|
| GUCY1A 2 | ROCK2 | AKT2 | GNAQ | ITGA2 | PIK3R2 | SYK | ITGB3 |
| GUCY1A 1 | ITGA2B | ARHGEF 12 | GNAS | GP5 | ITPR1 | VWF | PIK3CD |
| MAPK13 | ADCY5 | PRKCI | BTK | GP6 | PIK3R6 | GP1BB | GNAI1 |
| PPP1CA | ADCY4 | PLA2G4 E | FERMT3 | GP9 | RAP1A | F2 | GNAI2 |
| PPP1CB | ADCY3 | F2R | GUCY1B 1 | APBB1I P | RAP1B | GP1BA | PTGS1 |
| MAPK14 | ADCY2 | PLA2G4 F | ACTG1 | FCGR2A | TBXA2R | PLCB4 | MYLK4 |
| MAPK11 | ADCY9 | PLA2G4 C | RASGRP 2 | COL3A1 | P2RY1 | P2RX1 | GNAI3 |
| COL1A2 | ADCY8 | PLA2G4 D | MYLK | PIK3CB | FCER1G | JMJD7-PLA2G4 B | PIK3CG |
| COL1A1 | ADCY7 | PLA2G4 A | PRKACG | PIK3CA | FGA | PLCB2 | GNA13 |
| MAPK12 | MYL12B | PLA2G4 B | RASGRP 1 | LCP2 | PRKG2 | PLCB3 | MAPK3 |
| STIM1 | ADCY6 | VASP | PLCG2 | ARHGEF 1 | PRKG1 | F2RL3 | MAPK1 |
| ORAI1 | ARHGAP 35 | MYL12A | PRKACB | SNAP23 | FGB | PLCB1 | FYN |
| TLN2 | PPP1CC | PRKCZ | PRKACA | PIK3R5 | P2RY12 | MYLK2 | |
| TLN1 | ACTB | VAMP8 | LYN | ITPR2 | FGG | MYLK3 | |
| ROCK1 | AKT3 | PIK3R1 | RHOA | ITPR3 | NOS3 | ITGB1 | |
| ADCY1 | AKT1 | TBXAS1 | PPP1R1 2A | PIK3R3 | PTGIR | SRC | |

| RIG-I-like receptor signaling pathway | | | | | | | |
|---|---|---|---|---|---|---|---|
| MAP3K1 | MAPK12 | CXCL8 | CASP10 | CHUK | MAVS | IFNA1 | IFNB1 |
| MAPK13 | CYLD | RELA | FADD | IFNA17 | TRAF6 | IFNA4 | IL12B |
| MAPK14 | SIKE1 | NLRX1 | RIPK1 | TRAF3 | PIN1 | IFIH1 | IL12A |
| ATG12 | MAPK10 | DDX3X | MAP3K7 | TRAF2 | IFNA10 | IKBKB | IKBKG |
| MAPK11 | IRF3 | TRADD | AZI2 | ISG15 | IFNA6 | MAPK9 | IKBKE |
| CXCL10 | TBKBP1 | TNF | IFNA13 | STING1 | IFNA5 | MAPK8 | ATG5 |
| NFKBIB | IFNK | TANK | IFNA14 | NFKB1 | IFNA8 | TBK1 | IFNA21 |
| IFNE | IRF7 | CASP8 | DDX58 | IFNW1 | IFNA7 | DHX58 | |
| NFKBIA | OTUD5 | TKFC | IFNA16 | RNF125 | IFNA2 | TRIM25 | |

| T cell receptor signaling pathway | | | | | | | |
|---|---|---|---|---|---|---|---|
| MAPK13 | TNF | PAK6 | MAP3K1 4 | VAV1 | CD28 | CD3D | CD40LG |
| BUB1B-PAK6 | CBLB | PAK5 | BCL10 | VAV2 | LCP2 | PPP3CA | PIK3CD |
| MAPK14 | NRAS | CARD11 | PPP3R1 | VAV3 | CD247 | PPP3CB | MALT1 |
| MAPK11 | PAK2 | MAP2K2 | RASGRP 1 | IL2 | SOS2 | PPP3CC | IKBKB |
| TEC | PAK1 | MAP2K1 | PPP3R2 | NFKBIB | NFKBIE | CTLA4 | MAPK9 |
| MAPK12 | AKT3 | CD8B2 | PLCG1 | IL5 | ITK | ICOS | MAPK8 |
| MAPK10 | AKT1 | NFKB1 | MAP2K7 | IL4 | LAT | CHUK | MAPK3 |
| CD4 | AKT2 | DLG1 | RHOA | NFKBIA | SOS1 | PDPK1 | MAPK1 |
| PDCD1 | GRAP2 | PIK3R1 | IL10 | PTPRC | GSK3B | FOS | HRAS |
| PTPN6 | MAP3K7 | CD8B | JUN | PIK3CB | PIK3R3 | CDC42 | FYN |
| RAF1 | PAK4 | CD8A | NFATC3 | PIK3CA | PIK3R2 | ZAP70 | IKBKG |
| RELA | PAK3 | PRKCQ | NFATC2 | CDK4 | CD3G | LCK | NCK1 |
| CSF2 | MAP3K8 | GRB2 | NFATC1 | IFNG | CD3E | KRAS | NCK2 |

| Th1 and Th2 cell differentiation | | | | | | | |
|---|---|---|---|---|---|---|---|
| IL4R | NFKBIB | LAT | DLL4 | STAT5B | ZAP70 | GATA3 | MAPK1 |
| JUN | IL5 | RELA | PPP3CA | JAG1 | LCK | RBPJ | IL12A |
| IL13 | IL4 | RBPJL | PPP3CB | JAG2 | IL2RA | IKBKB | JAK3 |
| NFATC3 | NFKBIA | MAML3 | PPP3CC | HLA-DRB5 | IL2RB | PPP3R1 | IKBKG |
| NFATC2 | MAPK12 | MAML2 | TBX21 | STAT1 | HLA-DPB1 | PPP3R2 | HLA-DMA |
| NFATC1 | MAPK10 | MAML1 | STAT6 | IFNGR1 | PRKCQ | MAPK9 | JAK1 |
| TYK2 | CD4 | CD3G | IL12RB 2 | IFNGR2 | HLA-DRB4 | HLA-DMB | STAT5A |
| RUNX3 | IFNG | IL2RG | HLA-DOB | CHUK | HLA-DRB3 | MAPK8 | JAK2 |
| MAPK13 | HLA-DRA | DLL1 | IL12RB 1 | STAT4 | HLA-DRB1 | MAPK3 | |
| IL2 | HLA-DQB1 | CD3E | HLA-DQA2 | FOS | NOTCH3 | HLA-DPA1 | |
| MAPK14 | CD247 | DLL3 | HLA-DOA | NFKB1 | NOTCH1 | PLCG1 | |
| MAPK11 | NFKBIE | CD3D | HLA-DQA1 | MAF | NOTCH2 | IL12B | |

| Toll-like receptor signaling pathway | | | | | | | |
|---|---|---|---|---|---|---|---|
| TICAM2 | TNF | MAP2K1 | CCL4L1 | TLR1 | TLR4 | LBP | PIK3CD |
| TICAM1 | AKT3 | IFNA17 | CCL4L2 | NFKBIA | TLR3 | FADD | IKBKB |
| MAPK13 | AKT1 | TIRAP | TBK1 | IL6 | CXCL8 | RIPK1 | MAPK9 |
| MAPK14 | AKT2 | TRAF3 | IL12B | PIK3CB | CD40 | STAT1 | MAPK8 |
| MAPK11 | SPP1 | NFKB1 | IL12A | IRF5 | CXCL9 | CHUK | CCL5 |
| CXCL10 | MAP3K7 | MYD88 | CD14 | PIK3CA | CCL3L1 | FOS | MAPK3 |
| CXCL11 | MAP3K8 | PIK3R1 | MAP2K6 | IRF3 | CCL3L3 | IFNA6 | CCL4 |
| MAPK12 | MAP2K4 | TRAF6 | IFNA21 | TLR9 | LY96 | IFNA5 | CCL3 |
| MAPK10 | IFNA13 | TAB2 | MAP2K7 | TLR8 | PIK3R3 | IFNA8 | IFNB1 |
| TOLLIP | IFNA14 | TAB1 | JUN | IRF7 | PIK3R2 | IFNA7 | RAC1 |
| CD86 | MAP2K2 | IFNAR1 | IRAK4 | TLR7 | CASP8 | IFNA2 | MAPK1 |
| RELA | MAP2K3 | IFNA10 | TLR2 | TLR6 | CTSK | IFNA1 | IKBKG |
| CD80 | IFNA16 | IFNAR2 | IL1B | TLR5 | IRAK1 | IFNA4 | IKBKE |

| Central carbon metabolism in cancer | | | | | | | |
|---|---|---|---|---|---|---|---|
| PDGFRB | SLC7A5 | PIK3R3 | LDHC | IDH1 | PKM | KRAS | PTEN |
| PDGFRA | PIK3CB | SLC1A5 | LDHB | NTRK3 | PFKM | LDHAL6 A | PIK3CD |
| PDHA2 | SCO2 | PIK3R2 | LDHA | MAP2K2 | PFKL | FGFR3 | HIF1A |
| G6PD | PIK3CA | GLS | AKT3 | NTRK1 | KIT | LDHAL6 B | MAPK3 |
| TIGAR | TP53 | HK1 | ERBB2 | MAP2K1 | PIK3R1 | FGFR2 | MAPK1 |
| PDHA1 | RAF1 | EGFR | MYC | PGAM1 | HKDC1 | PFKP | HRAS |
| SIRT6 | RET | HK3 | AKT1 | IDH2 | FGFR1 | FLT3 | PDK1 |
| SIRT3 | GLS2 | HK2 | AKT2 | PGAM2 | PGAM4 | SLC2A1 | |
| GCK | PDHB | NRAS | SLC16A 3 | MTOR | MET | SLC2A2 | |

| Endometrial cancer | | | | | | | |
|---|---|---|---|---|---|---|---|
| EGF | BAX | PIK3R3 | AKT3 | TCF7L1 | DDB2 | PIK3CD | HRAS |
| BAD | SOS2 | PIK3R2 | CDH1 | MLH1 | PIK3R1 | ELK1 | CTNNA3 |
| AXIN2 | TP53 | ARAF | ERBB2 | APC2 | TCF7 | CTNNA2 | |
| AXIN1 | RAF1 | FOXO3 | MYC | GADD45 B | KRAS | MAPK3 | |
| BRAF | SOS1 | EGFR | AKT1 | MAP2K1 | GRB2 | POLK | |
| APC | LEF1 | NRAS | AKT2 | PDPK1 | CDKN1A | CTNNA1 | |
| PIK3CB | GSK3B | CASP9 | MAP2K2 | GADD45 A | CTNNB1 | MAPK1 | |
| PIK3CA | ILK | CCND1 | TCF7L2 | GADD45 G | PTEN | BAK1 | |

| PD-L1 expression and PD-1 checkpoint pathway in cancer | | | | | | | |
|---|---|---|---|---|---|---|---|
| MAP3K3 | EML4 | CD247 | CD3E | MAP2K2 | TIRAP | KRAS | HRAS |
| EGF | MAPK11 | PDCD1 | EGFR | MAP2K3 | NFKB1 | CD274 | IKBKG |
| JUN | TLR2 | PTPN6 | CD3D | STAT1 | MTOR | PTEN | JAK1 |
| NFATC3 | NFKBIB | NFKBIE | PPP3CA | CSNK2A 2 | MYD88 | PIK3CD | MAP2K6 |
| PTPN11 | NFKBIA | TLR4 | NRAS | IFNGR1 | ZAP70 | HIF1A | JAK2 |
| NFATC2 | MAPK12 | RAF1 | PPP3CB | CSNK2A 1 | LCK | IKBKB | |
| NFATC1 | CD4 | LAT | PPP3CC | MAP2K1 | PIK3R1 | PPP3R1 | |
| TICAM2 | PIK3CB | ALK | AKT3 | IFNGR2 | RPS6KB 2 | RASGRP 1 | |
| TICAM1 | PIK3CA | RELA | AKT1 | CHUK | RPS6KB 1 | PPP3R2 | |
| BATF | IFNG | PIK3R3 | AKT2 | STAT3 | CSNK2B | MAPK3 | |
| MAPK13 | TLR9 | PIK3R2 | BATF2 | CSNK2A 3 | TRAF6 | PLCG1 | |
| MAPK14 | CD28 | CD3G | BATF3 | FOS | PRKCQ | MAPK1 | |

| Transcriptional misregulation in cancer | | | | | | | |
|---|---|---|---|---|---|---|---|
| LMO2 | CCNT2 | IGFBP3 | CSF1R | PAX5 | BCL6 | KDM6A | FLT1 |
| IL1R2 | PRCC | TRAF1 | GRIA3 | HMGA2 | FEV | H3-3B | CDKN1B |
| ZBTB17 | CSF2 | GADD45 G | SLC45A 3 | NFKBIZ | ID2 | H3C8 | CEBPE |
| DEFA5 | HPGD | PBX3 | MAX | ETV1 | ATM | H3C7 | HDAC1 |
| DEFA6 | CCNT1 | ASPSCR 1 | SPI1 | PAX7 | REL | ARNT2 | FLT3 |
| ZBTB16 | EWSR1 | IGF1 | DDX5 | MMP9 | RARA | CDKN2C | CDKN1A |
| DEFA3 | MPO | NFKB1 | KMT2A | ETV4 | DOT1L | TAF15 | HDAC2 |
| RUNX1 | FOXO1 | FLI1 | JMJD1C | BAIAP3 | CXCL8 | PTCRA | LDB1 |
| IGH | HOXA11 | PBX1 | PDGFA | ETV5 | H3-3A | EYA1 | SIX1 |
| MITF | HOXA10 | PTK2 | PLAU | MLF1 | CD40 | NTRK1 | ITGAM |
| DEFA4 | RXRA | NR4A3 | PLAT | PAX3 | BCL2A1 | MEF2C | LYL1 |
| DEFA1 | HOXA9 | MAF | IGF1R | ETV6 | TMPRSS 2 | GZMB | HHEX |
| PML | RXRB | CCNA2 | ELK4 | TGFBR2 | MLLT1 | KLF3 | FCGR1A |
| RUNX2 | CCND2 | CCNA1 | TSPAN7 | IL3 | MLLT3 | DDB2 | H3C14 |
| WT1 | NSD2 | TLX3 | CD14 | WNT16 | BMI1 | H3-4 | H3C15 |
| MYCN | MYC | TLX1 | PROM1 | SUPT3H | AFF1 | PER2 | SIN3A |
| NUPR1 | RXRG | BCL2L1 | MEN1 | ETV7 | FUT8 | H3-5 | H3C12 |
| SPINT1 | RUNX1T 1 | ERG | SSX1 | ZEB1 | TFE3 | IL2RB | SIX4 |
| BAX | ELANE | COMMD3 -BMI1 | SSX2 | CDK9 | H3C2 | PPARG | H3C13 |
| CDK14 | NGFR | BMP2K | SS18 | NCOR1 | H3C1 | DDIT3 | POLK |
| TP53 | GADD45 B | TCF3 | DUSP6 | SP1 | DEFA1B | MET | H3C10 |
| CD86 | BCL11B | BIRC2 | FUS | IL6 | H3C4 | SSX2B | H3C11 |
| RELA | GADD45 A | BIRC3 | JUP | MEIS1 | H3C3 | CEBPA | BAK1 |
| ATF1 | SMAD1 | MDM2 | MMP3 | PAX8 | H3C6 | CEBPB | ITGB7 |

In the present invention, the immune cell composition is obtained by calculating a proportion of two or more immune cells selected from the group consisting of: naive CD4+ T cells, CD4+ T cells, naive CD8+ T cells, CD8+ T cells, central memory T cells, cytotoxic T cells, dendritic cells, effector memory T cells, exhausted T cells, γδ T cells, natural Treg cells, induced Treg cells, mucosal-associated invariant T cells, NK cells, NK T cells, follicular helper T cells, type 1 helper T cells, type 17 helper T cells, type 2 helper T cells, type 1 treg T cells, monocytes, macrophages, neutrophils, B cells, and infiltration scores.

In the present invention, the immune cell receptor features and transcriptome features in step (c) may include the features shown in Table 1 below.

**[Table 1]**

| | Feature List | |
|---|---|---|
| Level | Feature type | Feature description |
| | | Total number of TCR sequences |
| | | Unique number of TCR sequences |
| | | Number of TCR clonotypes per kilo-reads |
| | | Entropy of TCR |
| | | Clonality of TCR |
| | Immune repertoi re properti es | Total number of BCR sequences |
| | | Unique number of BCR sequences |
| | | Number of BCR clonotypes per kilo-reads |
| | | Entropy of BCR |
| | | Clonality of BCR |
| | | Somatic hypermutation rate of BCR sequences |
| | | Number of BCR clusters |
| CDR3 sequenc e | | IGH |
| | | IGK |
| | | IGL |
| | | TRA |
| | | TRB |
| | | TRD |
| | | TRG |
| | V&J gene usage, proporti on | IGHA1 |
| | | IGHA2 |
| | | IGHD |
| | | IGHE |
| | | IGHG1 |
| | | IGHG2 |
| | | IGHG3 |
| | | IGHG4 |
| | | IGHGP |
| | | IGHM |
| | | Mean of length in TCR |
| | | Mean of gravy index in TCR |
| | | Mean of bulkiness of amino acids in TCR |
| | | Mean of polarity of amino acids in TCR |
| | | Mean of net charge in TCR |
| | | Mean of fraction of basic amino acids in TCR |
| | | Mean of fraction of acidic amino acids in TCR |
| | | Mean of fraction of aromatic amino acids in TCR |
| | Chemical properti es of amino acids | Mean of aliphatic index in TCR |
| | | Mean of length in BCR |
| | | Mean of gravy index in BCR |
| | | Mean of bulkiness of amino acids in BCR |
| | | Mean of polarity of amino acids in BCR |
| | | Mean of net charge in BCR |
| | | Mean of fraction of basic amino acids in BCR |
| | | Mean of fraction of acidic amino acids in BCR |
| | | Mean of fraction of aromatic amino acids in BCR |
| | | Mean of aliphatic index in BCR |
| | | T cell activation involved in immune response |
| | | Innate immune response |
| | | Innate immune response activating cell surface receptor signaling pathway |
| | | Lymphocyte activation involved in immune response |
| | | Mucosal immune response |
| Transcr ipt | Single sample gene set enrichme nt analysis , ssGESA score | Natural killer cell activation involved in immune response |
| | | Negative regulation of immune response |
| | | Negative regulation of innate immune response |
| | | Neutrophil activation involved in immune response |
| | | Positive regulation of cytokine |
| | | Positive regulation of immune effector process |
| | | Positive regulation of immune response |
| | | Positive regulation of production of molecular mediator of immune response |
| | | Regulation of adaptive immune response |
| | | Regulation of cytokine production involved in immune response |
| | | Regulation of humoral immune response |
| | | Regulation of immune effector process |
| | | Regulation of immune response |
| | | Regulation of innate immune response |
| | | Antigen processing and presentation |
| | | B cell receptor signaling pathway |
| | | Chemokine signaling pathway |
| | | Complement and coagulation cascades |
| | | Cytosolic DNA-sensing pathway |
| | | Fc epsilon RI signaling pathway |
| | | Fc gamma R-mediated phagocytosis |
| | | Hematopoietic cell lineage |
| | | IL-17 signaling pathway |
| | | Intestinal immune network for IgA production |
| | | Leukocyte transendothelial migration |
| | | NOD-like receptor signaling pathway |
| | | Natural killer cell mediated cytotoxicity |
| | | Neutrophil extracellular trap formation |
| | | Platelet activation |
| | | RIG-I-like receptor signaling pathway |
| | | T cell receptor signaling pathway |
| | | Th1 and Th2 cell differentiation |
| | | Toll-like receptor signaling pathway |
| | | Cellular response to tumor necrosis factor |
| | | Negative regulation of tumor necrosis factor production |
| | | Negative regulation of tumor necrosis factor superfamily cytokine production |
| | | Negative regulation of tumor necrosis factor-mediated signaling pathway |
| | | Positive regulation of tumor necrosis factor production |
| | | Positive regulation of tumor necrosis factor superfamily cytokine production |
| | | Regulation of tumor necrosis factor production |
| | | Regulation of tumor necrosis factor-mediated signaling pathway |
| | | Response to tumor necrosis factor |
| | | Tumor necrosis factor-mediated signaling pathway |
| | | Central carbon metabolism in cancer |
| | | Endometrial cancer |
| | | PD-L1 expression and PD-1 checkpoint pathway in cancer |
| | | Transcriptional misregulation in cancer |
| | | naive CD4+ T cell |
| | | CD4+ T cell |
| | | naive CD8+ T cell |
| | | CD8+ T cell |
| | | Central memory T cell |
| | | Cytotoxic T cell |
| | | Dendritic cell |
| | | Effector memory T cell |
| | | Exhausted T cell |
| | | γδ T cell |
| | | Natural Treg cell |
| | | Induced Treg cell |
| | Immune cell composit ion | Mucosal-associated invariant T cell |
| | | NK cell |
| | | NK T cell |
| | | Follicular helper T cell |
| | | Type 1 helper T cell |
| | | Type 17 helper T cell |
| | | Type 2 helper T cell |
| | | Type 1 Treg cell |
| | | Monocyte |
| | | Macrophage |
| | | Neutrophil |
| | | B cell |
| | | Infiltration score |

In the present invention, the cut-off value in step (d) may be used without limitation as long as it is a value used to distinguish between a cancer patient and a normal subject, and may be preferably 0.5, but is not limited thereto. When the cut-off value is 0.5, a case with an output value of 0.5 or more is determined that there is cancer.

In the present invention, when the artificial intelligence model was trained to label a cancer patient as 1 and a normal sample as 0, so that the output value can be interpreted as the probability of having cancer. Based on this result, when the probability of having cancer is 0.5 or higher, the patient may be diagnosed as having cancer, and when the probability of having cancer is 0.5 or lower, the patient may be diagnosed as normal.

Here, it will be apparent to those skilled in the art that the cut-off value of 0.5 may be arbitrarily changed. For example, in an attempt to reduce false positives, the cut-off value may be set to be higher than 0.5 as a stricter criterion for determining whether or not there is cancer, and in an attempt to reduce false negatives, the cut-off value may be set to be lower than 0.5 as a weaker criterion for determining that there is cancer.

According to the present invention, the method may further include:
(e) inputting the immune cell receptor features and transcriptome features of the sample primarily determined that there is cancer into a second artificial intelligence model trained to distinguish between an immunoactive state and cancer, comparing the analyzed output value with a cut-off value and finally determining whether or not there is cancer.

In the present invention, the immunoactive state means a state in which the overall immune system is activated due to reasons such as an immune-related disease other than cancer or inflammation, and the calculated immune cell receptor features and transcriptome features are similar to those of cancer patients, and the step (e) of the present invention means training to distinguish between a normal sample of the immunoactive state and a cancer sample, to distinguish the immunoactive state as normal and the cancer sample as cancer.

It is well known that the immunological profiling of normal subjects in immune-related diseases or inflammation states is similar to that of cancer patients (Zeev Elkoshi, Front. Immunol., Vol. 13:821598, 2022; Giat E. et al., Autoimmunity Reviews, Vol. 16(10), pp. 1049-1057, 2017).

In the present invention, the cut-off value in step (d) may be used without limitation as long as it is a value used to distinguish between a cancer patient and a normal subject, and may be preferably 0.5, but is not limited thereto. When the cut-off value is 0.5, a case having an output value of 0.5 or more is determined that there is cancer.

In the present invention, the artificial intelligence model may be used without limitation as long as it is a model trained to distinguish between cancer and normality, and preferably includes at least one selected from the group consisting of a linear regression, a random forest, a convolutional neural network (CNN), a deep neural network (DNN), a recurrent neural network (RNN), and an autoencoder, but is not limited thereto.

In the present invention, when the artificial intelligence model is a deep neural network, the loss function may be represented by the following Equation 1:

$BCE = - {\sum }_{i = 0}^{N} \left[{y}_{i} log\left(\hat{{y}_{i}}\right)+\left(1-{y}_{i}\right)log\left(1-\hat{{y}_{i}}\right)\right]$
wherein N is the total number of samples, *yᵢ* is the actual label of the i^{th} input value, and $\hat{{y}_{i}}$ is the label of the i^{th} input value predicted by the model.

In the present invention, when the artificial intelligence model is a DNN, the training includes the following steps:
i) classifying the produced transcriptome data into training, validation, and test data,
   wherein the training data is used to train the model and validate hyper-parameter tuning, and the test data is used for the test after optimal model production; and
ii) evaluating the performance of the optimal model built using test data using various indicators and analyze the feature importance of the model.

In the present invention, the hyper-parameter tuning is a process of optimizing the values of various parameters (such as the number of convolution layers, the number of dense layers, and the number of convolution filters) constituting the artificial intelligence model. Hyper-parameter tuning is performed using Bayesian optimization, manual search or grid search.

In the present invention, the internal parameters (weights) of the artificial intelligence model are optimized using predetermined hyper-parameters, and model training is stopped before the training loss stops in order to prevent overfitting.

In the present invention, the output value obtained by analyzing immune cell receptor features and transcriptome features input by the artificial intelligence model in step (d) or (e) may be used without limitation, as long as it is a specific score or real number, and the value is preferably a real number, but is not limited thereto.

In the present invention, when the artificial intelligence model is a DNN, the real number means a value expressed as a probability value by adjusting the output of the artificial intelligence model to a scale of 0 to 1 using applying the sigmoid function or SoftMax function for the last layer.

In another aspect, the present invention is directed to a device for diagnosing cancer based on immunological profiling, the device including:
a decoder configured to decode whole transcriptome sequencing (WTS) information from a biological sample;
an aligner configured to align the decoded WTS information to a reference genome database;
a feature detector configured to extract immune cell receptor features and transcript features from the aligned WTS information; and
a cancer diagnostic unit configured to compare the analyzed output obtained by inputting the extracted features to an artificial intelligence model trained to distinguish between a cancer patient and a normal subject, with a cut-off value, and determine whether or not cancer is present.

In the present invention, the decoder may include a nucleic acid injector configured to inject the nucleic acid extracted from an independent device, and a sequence information analyzer configured to analyze the sequence information of the injected nucleic acid, preferably an NGS analyzer, but is not limited thereto.

In the present invention, the decoder may receive and decode sequence information data generated in the independent device.

In another aspect,
the present invention is directed to a computer-readable storage medium including an instruction configured to be executed by a processor for providing information for diagnosis of cancer, through the following steps including:
(a) obtaining whole transcriptome sequencing (WTS) information from a biological sample;
(b) aligning the obtained WTS information to a reference genome database;
(c) extracting immune cell receptor features and transcriptome features from the aligned WTS information;
(d) inputting the extracted features to an artificial intelligence model trained to distinguish between a cancer patient and a normal subject, comparing the analyzed output value with a cut-off value, and primarily determining whether or not there is cancer; and
(e) inputting the immune cell receptor features and transcriptome features of the sample primarily determined that there is cancer into a second artificial intelligence model trained to distinguish between an immunoactive state and cancer, comparing the analyzed output value with a cut-off value, and finally determining whether or not there is cancer.

In another aspect, the method according to the present disclosure may be implemented using a computer. In one embodiment, the computer includes one or more processors coupled to a chipset. In addition, a memory, a storage device, a keyboard, a graphics adapter, a pointing device, a network adapter and the like are connected to the chipset. In one embodiment, the performance of the chipset is acquired by a memory controller hub and an I/O controller hub. In another embodiment, the memory may be directly coupled to a processor instead of the chipset. The storage device is any device capable of maintaining data, including a hard drive, compact disc read-only memory (CD-ROM), DVD, or other memory devices. The memory relates to data and instructions used by the processor. The pointing device may be a mouse, track ball or other type of pointing device, and is used in combination with a keyboard to transmit input data to a computer system. The graphics adapter presents images and other information on a display. The network adapter is connected to the computer system through a local area network or a long distance communication network. However, the computer used herein is not limited to the above configuration, may not have some configurations, may further include additional configurations, and may also be part of a storage area network (SAN), and the computer of the present invention may be configured to be suitable for the execution of modules in the program for the implementation of the method according to the present invention.

The module used herein may mean a functional and structural combination of hardware to implement the technical idea according to the present invention and software to drive the hardware. For example, it is apparent to those skilled in the art that the module may mean a logical unit of predetermined code and a hardware resource to execute the predetermined code, and does not necessarily mean physically connected code or one type of hardware.

### Example

Hereinafter, the present invention will be described in more detail with reference to examples. However, it will be obvious to those skilled in the art that these examples are provided only for illustration of the present invention, and should not be construed as limiting the scope of the present invention.

### Example 1. Extracting RNA from blood and performing next-generation sequencing

10 mL of blood was collected from 72 normal people and 131 cancer patients (15 colon cancer patients, 18 esophageal cancer patients, 21 liver cancer patients, 42 lung cancer patients, 14 ovarian cancer patients, and 21 pancreatic cancer patients) and stored in blood storage containers (Streck tubes). After collection, the blood was centrifuged at 3,000 rpm for 10 minutes within 24 hours, and the buffy coat was collected and used for RNA extraction. RNA was extracted using RiboPure^{™} RNA Purification Kit (ThermoFisher scientific, USA), mRNA was isolated using Dynabeads mRNA purification kit (ThermoFisher scientific, USA), and then subjected to RNA fragmentation, a library was produced using xGen broad-range RNA library prep kit (IDT, USA), and sequenced using Novaseq 6000 system (illumina, USA) equipment at 200 cycles.

The result showed that an average of 90 million reads were produced for each sample.

The patient composition is shown in Table 2 below.

**[Table 2]**

| Type of cancer | Number of patients | Type of cancer | Number of patients | Type of cancer | Number of patients |
|---|---|---|---|---|---|
| Colon cancer | 15 | Esophageal cancer | 18 | Liver cancer | 21 |
| Lung cancer | 42 | Ovarian cancer | 14 | Pancreatic cancer | 21 |
| Normal | 72 | | | | |

### 1-2. Acquisition of mass data for model construction and validation

To produce a cancer diagnosis model, 10,929 cancer and 10,845 normal tissue sample WTS data were collected from the cancer genome atlas (TCGA) and the genotype-tissue expression (GTEx) consortium, respectively.

In addition, for model optimization, whole blood WTS data from 356 immunological disease (inflammatory bowel disease (IBD), juvenile idiopathic arthritis (JIA), and COVID19 patients were obtained from GSE190680, GSE179627, and GSE112057 in gene expression omnibus.
Source: TCGA (https://portal.gdc.cancer.gov/)
GTEx (https://gtexportal.org/)
GEO (https://www.ncbi.nlm.nih.gov/geo/)

### 1-3. Pretreatment of acquired sequence information

TCR/BCR sequences were extracted from the bam files of the aligned samples obtained in Example 1-1 using the TRUST4 tool. The TRUST4 tool is an algorithm that obtains and analyzes TCR/BCR sequences including the CDR3 region from RNA sequencing data through de novo assembly (https://github.com/liulab-dfci/TRUST4). At this time, samples having three or less sequences were excluded and sequences containing N or having a length greater than 20 and less than 12 were excluded.

### Example 2. Extraction of immune cell receptor features and transcriptome features for cancer diagnosis

### 2-1. Extraction of immune cell receptor features

From the extracted CDR3 sequence data, the TCR/BCR repertoire features of the CDR3 sequences extracted by TRUST4 into the R package called "RIMA" (Aashna Jhaveri et al., RIMA_pipeline, (2021), GitHub repository, https:/github.com/liulabdfci/RIMA_pipeline) were calculated for each of TCR and BCR. The number of unique CDR3 sequences was divided by the total number and multiplied by 1000 to calculate CPK, and the remaining features were calculated using the basic function provided by RIMA, and used as features of the AI model. The entire features related to the immune repertoire are described in the immune repertoire features in Table 1.

In addition, the V and J gene usages were calculated from the extracted CDR3 sequence data and used as features of the artificial intelligence model. The genotype ratios of IGH, IGK, IGL, TRA, TRB, TRD, TRG, IGHA1, IGHA2, IGHD, IGHE, IGHG1, IGHG2, IGHG3, IGHG4, IGHGP, and IGHM were calculated for each sample using the following Equation 2. $\begin{matrix}gene usage of genotype i = \\ \frac{number of CDR 3 sequences corresponding to genotype i in the V & J regions}{total number of CDR 3 sequences}\end{matrix}$

In addition, the chemical properties of the CDR3 amino acid sequence were calculated using the R package "alakazam" (Namita T. et al., Bioinformatics, Vol.31, No. 20, pp. 3356-3358, 2015) and were used as features of the artificial intelligence model. The overall features of the chemical properties of the CDR3 amino acid sequence are described in the chemical properties of the amino acid sequence in Table 1.

### 2-2. Extraction of transcriptome features

The bam file of the WTS data obtained in Example 1 was converted into a gene expression matrix using the featureCounts command of the algorithm in order to use the bam file in the ImmuCellAI (YR Miao et al., Adv. Sci. 7:1902880, 2020) algorithm. Then, TPM normalization was performed. That is, the read count of each gene was divided by the length of the transcript and then the result was divided again by the sum of the corrected values, so that the total TPM of each sample was adjusted to 1.

In the normalized gene expression matrix, the ensemble gene ID was converted to HGNC gene name, duplicated genes were removed, and then 24 immune cell compositions (immune cell composition) and 1 immune filtration score were calculated using the ImmuCellAI basic function and used as features of the AI model. The overall features related to the composition of immune cells are described in the immune cell composition in Table 1.

In addition, the enrichment scores of a total of 52 gene sets related to immune response (38) or cancer (14) were calculated using single sample gene set enrichment analysis (ssGSEA, https://gseapy.readthedocs.io/en/latest/introduction.html) and used as features of the AI model. The overall features of the scores are described in the cancer- or immune-related gene enrichment scores in Table 1.

Specifically, among the gene sets related to immune response or cancer, GO terms were selected from https://www.ebi.ac.uk/QuickGO/annotations, KEGG pathway was selected from https://www.kegg.jp/kegg/pathway.html, and the gene list for each gene set is shown in Table 5 above. The TPM normalization matrix and the selected gene set list were input and the features were calculated using the GSEApy basic function.

### Example 3. Construction of cancer diagnosis model and training process

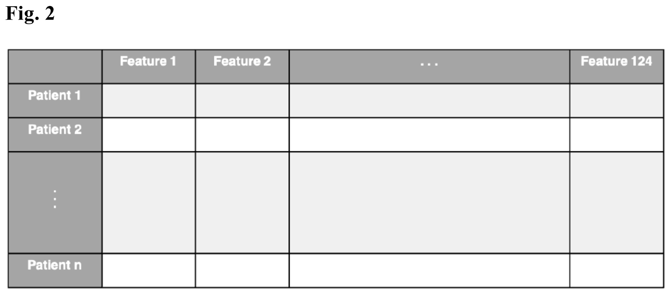

The features extracted in Example 2 were created as input matrixes as shown in FIG. 2 and used to construct a cancer diagnosis model.

The data sets used for training were 10,929 cancer and 10,845 normal tissue sample WTS data from the cancer genome atlas (TCGA) and the genotype-tissue expression (GTEx) consortium obtained in Example 1-2, respectively, and were divided into training set, validation set, and test set as shown in Table 3 below and used as learning and internal test set.

**[Table 3]**

| | Train | Validation | Test |
|---|---|---|---|
| TCGA | 9345 | 500 | 1000 |
| GTEx | 9429 | 500 | 1000 |

A binary classification model to distinguish between cancer patients and normal subjects was constructed using the AI algorithm. Three AI models were used for model training: linear regression, random forest, and deep neural network (DNN).

In order to optimize the linear regression and random forest models, the training data was repeatedly classified into training data and validation data using 5-fold cross validation, and the hyper-parameter tuning process was performed.

The DNN included an input layer that recognizes each feature of the input matrix as an independent node, three hidden layers, and an output layer that calculates the cancer score. Only the output layer used the sigmoid function as an activation function to calculate the final cancer score, and the remaining layers used the Relu function as an activation function.

The loss function of DNN used herein was binary crossentropy represented by Equation 1 and the loss function optimization was performed using the ADAM (Adaptive Moment Estimation) algorithm (Goodfellow, I., Bengio, Y., & Courville, A. (2016). Deep Learning. MIT Press).

The validation loss was monitored during model training using the basic function called "EarlyStopping" built in Keras (https://keras.io). When the training was not improved, it was stopped and the final performance evaluation was performed.

### Example 4. Performance verification of constructed cancer diagnosis model

An input matrix consisting of 124 features was created for the three models trained using the validation set described in Table 3 of Example 3, and the model performance was evaluated by receiver operating characteristic (ROC) curve analysis, and prediction accuracy, sensitivity, and specificity.

The result showed, as shown in B of FIG. 3, that the DNN model properly distinguished cancer samples from normal samples in all data sets, and as can be seen from A of FIG. 3, the DNN model exhibited superior AUC, accuracy, sensitivity, and specificity in all data sets.

**[Table 4]**

| | Validation |
|---|---|
| ROC-AUC | 0.99 |
| PR-AUC | 0.98 |

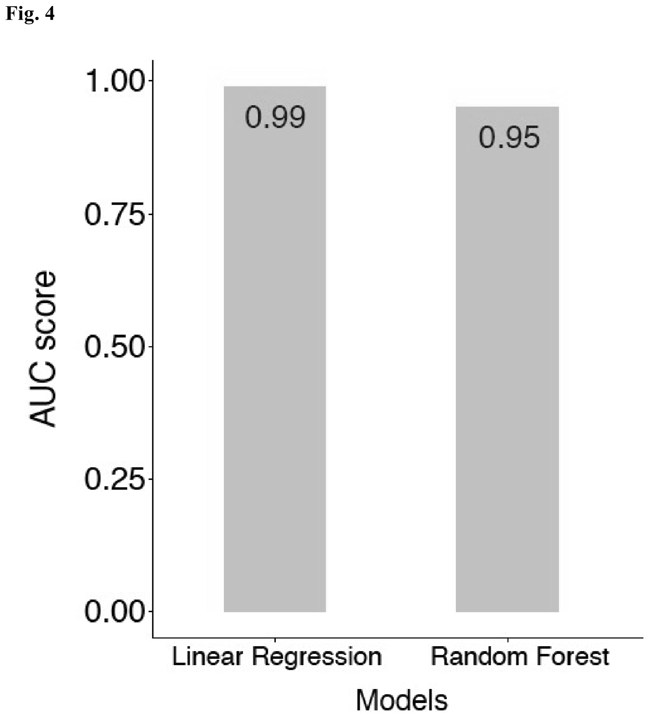

On the other hand, as can be seen from FIG. 4, the linear regression and random forest models exhibited high performance in tissue sample data, but significantly lower prediction performance in whole blood samples.

### Example 5. Analysis of important features of cancer diagnosis model

Which feature among the 124 features used in training the algorithm had an important role in performing cancer diagnosis was determined.

The SHAP algorithm of Python (https://shap.readthedocs.io/en/latest/) can be used to calculate which features are given greater weight when the model is trained. Based thereon, which features among the features calculated from the WTS data have an effect on cancer diagnosis was determined.

Specifically, the Shapley value indicates how much each feature contributed to the model predicting the result, and is the sum of the differences between the average of the predicted values from all possible combinations when a specific feature is excluded and the predicted values when the corresponding feature is added to each combination. The model trained in Example 3, the input values used for training were input into the built-in function called "DeepExplainer" of the SHAP package to calculate the Shapley value, and then the Shapley value calculated for each sample was converted into a matrix and visualized as a heatmap using R Studio.

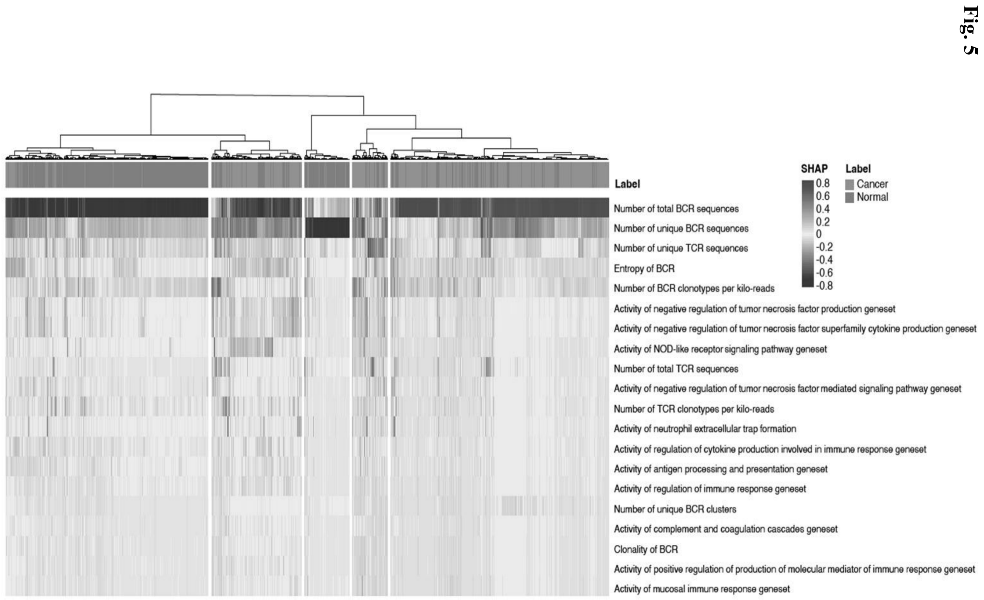

The result showed that, as shown in FIG. 5, among the features related to the immune repertoire, features related to BCR were at the top.

### Example 6. Additional model construction and performance verification for false positive removal

### 6-1. Model construction

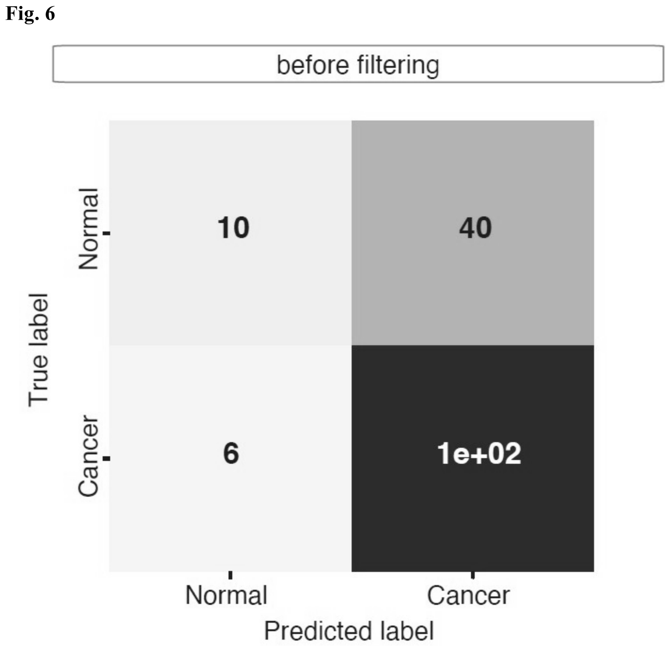

The input matrix consisting of 124 features from the WTS data of 183 cancer patients obtained in Example 1-1 was input into the DNN model of Example 3 and then output. The result showed, among the samples determined as cancer patients, many immunoactive normal samples were found as false positives (FIG. 6).

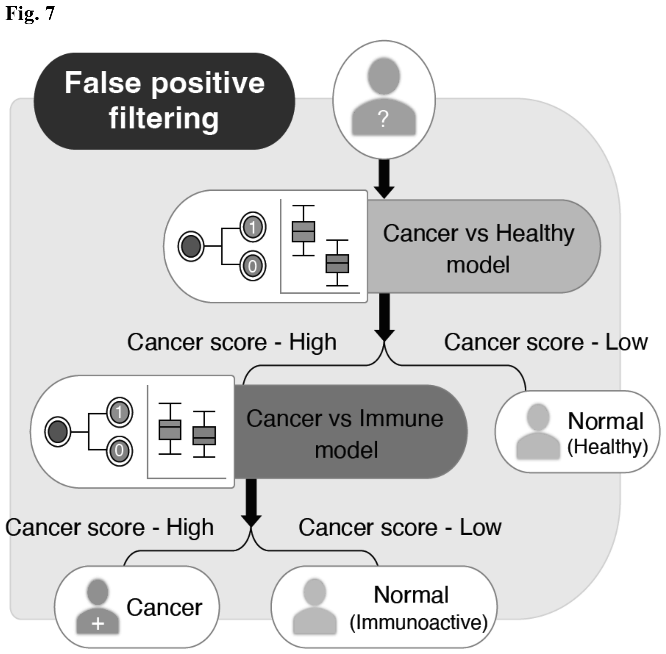

Therefore, an additional model capable of distinguishing between an immunoactive state and cancer was constructed (FIG. 7). The input matrix was the same as Example 3, but the training data used herein was the WTS data of 356 immunological disease patients' whole blood obtained in Example 1-2 and the normal whole blood data used for model verification, and the same training process as in Example 3 was performed.

### 6-2. Verification of performance of constructed false positive removal model

The performance of the DNN model of Example 3, to which the model constructed in Example 6-1 was added, was verified by creating an input matrix consisting of 124 features from the WTS data of 183 cancer patients obtained in Example 1-1. The result showed that the performance of the DNN model with the false positive removal model added was improved, as can be seen from FIG. 8.

### [Industrial applicability]

The method of diagnosing cancer based on immunological profiling using transcriptomes according to the present invention provides diagnosis of all cancer types based on artificial intelligence using both the features of immune cell receptors and the features of gene expression levels extracted from transcriptome samples produced by next-generation sequencing (NGS) and is highly commercially applicable due to the high accuracy and sensitivity thereof compared to diagnostic methods using only immune cell receptor sequence information.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

## Claims

1. A method of providing information for cancer diagnosis based on immunological profiling using transcriptomes, the method comprising:
(a) obtaining whole transcriptome sequencing (WTS) information from a biological sample;
(b) aligning the obtained WTS information to a reference genome database;
(c) extracting immune cell receptor features and transcriptome features from the aligned WTS information; and
(d) inputting the extracted features to an artificial intelligence model trained to distinguish between a cancer patient and a normal subject, and comparing the analyzed output value with a cut-off value to determine whether or not there is cancer.

2. A method of diagnosing cancer diagnosis based on immunological profiling using transcriptomes, the method comprising:
(a) obtaining whole transcriptome sequencing (WTS) information from a biological sample;
(b) aligning the obtained WTS information to a reference genome database;
(c) extracting immune cell receptor features and transcriptome features from the aligned WTS information; and
(d) inputting the extracted features to an artificial intelligence model trained to distinguish between a cancer patient and a normal subject and comparing the analyzed output value with a cut-off value to determine whether or not there is cancer.

3. The method according to claim 1 or 2, wherein the obtaining whole transcriptome sequencing (WTS) information from a biological sample in step (a) comprises:
(a-i) randomly fragmenting RNA obtained from a biological sample using an enzymatic cleavage method;
(a-ii) replacing the cleavage RNA with cDNA and then producing a library; and
(a-iii) performing single-end sequencing or pair-end sequencing on the produced library using a next-generation sequencer to obtain WTS information.

4. The method according to claim 1 or 2, wherein the step (c) of extracting the immune cell receptor features comprises:
(c-i) extracting a CDR3 (complementary-determining region 3) amino acid sequence of the immune cell receptor from the aligned WTS information; and
(c-ii) calculating at least one feature selected from the group consisting of immune repertoire properties, V&J gene usage, and chemical features of CDR3 amino acid sequences based on the extracted sequence information.

5. The method according to claim 1 or 2, wherein the step (c) of extracting transcriptome features comprises calculating a cancer- or immune-related gene enrichment score (single sample gene set enrichment analysis, ssGESA score) or an immune cell composition.

6. The method according to claim 4, wherein the immune repertoire properties comprise at least one selected from the group consisting of a total number of TCR sequences, a unique number of TCR sequences, a number of TCR clonotypes per kilo-reads, an entropy of TCR, clonality of TCR, a total number of BCR sequences, a unique number of BCR sequences, a number of BCR clonotypes per kilo-reads, an entropy of BCR, clonality of BCR, a somatic hypermutation rate of BCR sequences, and a number of BCR clusters.

7. The method according to claim 4, wherein the V&J gene usage is a proportion of two or more genotypes selected from the group consisting of IGH, IGK, IGL, TRA, TRB, TRD, TRG, IGHA1, IGHA2, IGHD, IGHE, IGHG1, IGHG2, IGHG3, IGHG4, IGHGP, and IGHM.

8. The method according to claim 4, wherein the chemical properties of the CDR3 amino acid sequence comprise at least one selected from the group consisting of a mean of length in TCR, a mean of gravy index in TCR, a mean of bulkiness of amino acids in TCR, a mean of polarity of amino acids in TCR, a mean of net charge in TCR, a mean of fraction of basic amino acids in TCR, a mean of fraction of acidic amino acids in TCR, a mean of fraction of aromatic amino acids in TCR, a mean of aliphatic index in TCR, a mean of length in BCR, a mean of gravy index in BCR, a mean of bulkiness of amino acids in BCR, a mean of BCR amino acids, a mean of polarity of amino acids in BCR, a mean of net charge in BCR, a mean of fraction of basic amino acids in BCR, a mean of fraction of acidic amino acids in BCR, a mean of fraction of aromatic amino acids in BCR, and a mean of aliphatic index in BCR.

9. The method according to claim 5, wherein the cancer- or immune-related gene enrichment score (single sample gene set enrichment analysis, ssGESA score) is obtained by calculating an enrichment score of a gene associated with at least one selected from the group consisting of T cell activation involved in immune response, innate immune response, innate immune response activating cell surface receptor signaling pathway, lymphocyte activation involved in immune response, mucosal immune response, natural killer cell activation involved in immune response, negative regulation of immune response, negative regulation of innate immune response, neutrophil activation involved in immune response, positive regulation of cytokine, production involved in immune response, positive regulation of immune effector process, positive regulation of immune response, positive regulation of production of molecular mediator of immune response, regulation of adaptive immune response, regulation of cytokine production involved in immune response, regulation of humoral immune response, regulation of immune effector process, regulation of immune response, regulation of innate immune response, antigen processing and presentation, B cell receptor signaling pathway, chemokine signaling pathway, complement and coagulation cascades, cytosolic DNA-sensing pathway, Fc epsilon RI signaling pathway, Fc gamma R-mediated phagocytosis, hematopoietic cell lineage, IL-17 signaling pathway, intestinal immune network for IgA production, leukocyte transendothelial migration, NOD-like receptor signaling pathway, natural killer cell mediated cytotoxicity, neutrophil extracellular trap formation, platelet activation, RIG-I-like receptor signaling pathway, T cell receptor signaling pathway, Th1 and Th2 cell differentiation, toll-like receptor signaling pathway, cellular response to tumor necrosis factor, negative regulation of tumor necrosis factor production, negative regulation of tumor necrosis factor superfamily cytokine production, negative regulation of tumor necrosis factor-mediated signaling pathway, positive regulation of tumor necrosis factor production, positive regulation of tumor necrosis factor superfamily cytokine production, regulation of tumor necrosis factor production, regulation of tumor necrosis factor-mediated signaling pathway, response to tumor necrosis factor, tumor necrosis factor-mediated signaling pathway, central carbon metabolism in cancer, endometrial cancer, PD-L1 expression and PD-1 checkpoint pathway in cancer, and transcriptional misregulation in cancer.

10. The method according to claim 5, wherein the immune cell composition is obtained by calculating a proportion of two or more immune cells selected from the group consisting of: naive CD4+ T cells, CD4+ T cells, naive CD8+ T cells, CD8+ T cells, central memory T cells, cytotoxic T cells, dendritic cells, effector memory T cells, exhausted T cells, γδ T cells, natural Treg cells, induced Treg cells, mucosal-associated invariant T cells, NK cells, NK T cells, follicular helper T cells, type 1 helper T cells, type 17 helper T cells, type 2 helper T cells, type 1 Treg T cells, monocytes, macrophages, neutrophils, B cells, and infiltration scores.

11. The method according to claim 1 or 2, wherein the immune cell receptor features and transcriptome features in step (c) comprise features shown in Table 1 below.
**[Table 1]**
| Feature List | | |
|---|---|---|
| Level | Feature type | Feature description |
| | | Total number of TCR sequences |
| | | Unique number of TCR sequences |
| | | Number of TCR clonotypes per kilo-reads |
| | | Entropy of TCR |
| | Immune repertoi re properti es | Clonality of TCR |
| | | Total number of BCR sequences |
| | | Unique number of BCR sequences |
| | | Number of BCR clonotypes per kilo-reads |
| CDR3 sequenc e | | Entropy of BCR |
| | | Clonality of BCR |
| | | Somatic hypermutation rate of BCR sequences |
| | | Number of BCR clusters |
| | | IGH |
| | | IGK |
| | V&J gene usage, proporti on | IGL |
| | | TRA |
| | | TRB |
| | | TRD |
| | | TRG |
| | | IGHA1 |
| | | IGHA2 |
| | | IGHD |
| | | IGHE |
| | | IGHG1 |
| | | IGHG2 |
| | | IGHG3 |
| | | IGHG4 |
| | | IGHGP |
| | | IGHM |
| | | Mean of length in TCR |
| | | Mean of gravy index in TCR |
| | | Mean of bulkiness of amino acids in TCR |
| | | Mean of polarity of amino acids in TCR |
| | | Mean of net charge in TCR |
| | | Mean of fraction of basic amino acids in TCR |
| | | Mean of fraction of acidic amino acids in TCR |
| | Chemical properti es of amino acids | Mean of fraction of aromatic amino acids in TCR |
| | | Mean of aliphatic index in TCR |
| | | Mean of length in BCR |
| | | Mean of gravy index in BCR |
| | | Mean of bulkiness of amino acids in BCR |
| | | Mean of polarity of amino acids in BCR |
| | | Mean of net charge in BCR |
| | | Mean of fraction of basic amino acids in BCR |
| | | Mean of fraction of acidic amino acids in BCR |
| | | Mean of fraction of aromatic amino acids in BCR |
| | | Mean of aliphatic index in BCR |
| Transcr ipt | Single sample gene set enrichme nt analysis | T cell activation involved in immune response |
| | | Innate immune response |
| | | Innate immune response activating cell surface receptor signaling pathway |
| | | Lymphocyte activation involved in immune response |
| | | Mucosal immune response |
| | , ssGESA score | Natural killer cell activation involved in immune response |
| | | Negative regulation of immune response |
| | | Negative regulation of innate immune response |
| | | Neutrophil activation involved in immune response |
| | | Positive regulation of cytokine |
| | | Positive regulation of immune effector process |
| | | Positive regulation of immune response |
| | | Positive regulation of production of molecular mediator of immune response |
| | | Regulation of adaptive immune response |
| | | Regulation of cytokine production involved in immune response |
| | | Regulation of humoral immune response |
| | | Regulation of immune effector process |
| | | Regulation of immune response |
| | | Regulation of innate immune response |
| | | Antigen processing and presentation |
| | | B cell receptor signaling pathway |
| | | Chemokine signaling pathway |
| | | Complement and coagulation cascades |
| | | Cytosolic DNA-sensing pathway |
| | | Fc epsilon RI signaling pathway |
| | | Fc gamma R-mediated phagocytosis |
| | | Hematopoietic cell lineage |
| | | IL-17 signaling pathway |
| | | Intestinal immune network for IgA production |
| | | Leukocyte transendothelial migration |
| | | NOD-like receptor signaling pathway |
| | | Natural killer cell mediated cytotoxicity |
| | | Neutrophil extracellular trap formation |
| | | Platelet activation |
| | | RIG-I-like receptor signaling pathway |
| | | T cell receptor signaling pathway |
| | | Th1 and Th2 cell differentiation |
| | | Toll-like receptor signaling pathway |
| | | Cellular response to tumor necrosis factor |
| | | Negative regulation of tumor necrosis factor production |
| | | Negative regulation of tumor necrosis factor superfamily cytokine production |
| | | Negative regulation of tumor necrosis factor-mediated signaling pathway |
| | | Positive regulation of tumor necrosis factor production |
| | | Positive regulation of tumor necrosis factor superfamily cytokine production |
| | | Regulation of tumor necrosis factor production |
| | | Regulation of tumor necrosis factor-mediated signaling pathway |
| | | Response to tumor necrosis factor |
| | | Tumor necrosis factor-mediated signaling pathway |
| | | Central carbon metabolism in cancer |
| | | Endometrial cancer |
| | | PD-L1 expression and PD-1 checkpoint pathway in cancer |
| | | Transcriptional misregulation in cancer |
| | | Naive CD4+ T cell |
| | | CD4+ T cell |
| | | Naive CD8+ T cell |
| | | CD8+ T cell |
| | | Central memory T cell |
| | | Cytotoxic T cell |
| | | Dendritic cell |
| | Immune cell composit ion | Effector memory T cell |
| | | Exhausted T cell |
| | | γδ T cell |
| | | Natural Treg cell |
| | | Induced Treg cell |
| | | Mucosal-associated invariant T cell |
| | | NK cell |
| | | NK T cell |
| | | Follicular helper T cell |
| | | Type 1 helper T cell |
| | | Type 17 helper T cell |
| | | Type 2 helper T cell |
| | | Type 1 Treg cell |
| | | Monocyte |
| | | Macrophage |
| | | Neutrophil |
| | | B cell |
| | | Infiltration score |

12. The method according to claim 1 or 2, wherein the cut-off value of step (d) is 0.5 and a case with an output value of 0.5 or higher is determined that there is cancer.

13. The method according to claim 1 or 2, further comprising:
(e) inputting the immune cell receptor features and transcriptome features of the sample primarily determined that there is cancer into a second artificial intelligence model trained to distinguish between an immunoactive state and cancer, comparing the analyzed output value with a cut-off value, and finally determining whether or not there is cancer.

14. The method according to claim 13, wherein the cut-off value of step (d) is 0.5 and a case with an output value of 0.5 or higher is determined that there is cancer.

15. The method according to claim 1, 2 or 13, wherein the artificial intelligence model comprises at least one selected from the group consisting of a linear regression, a random forest, a convolutional neural network (CNN), a deep neural network (DNN), a recurrent neural network (RNN), and an autoencoder.

16. The method according to claim 15, wherein when the artificial intelligence model is a deep neural network, the loss function is represented by the following Equation 1: $BCE = - {\sum }_{i = 0}^{N} \left[{y}_{i} log\left(\hat{{y}_{i}}\right)+\left(1-{y}_{i}\right)log\left(1-\hat{{y}_{i}}\right)\right]$ wherein N is a total number of samples, *yᵢ* is an actual label of an i^{th} input value, and $\hat{{y}_{i}}$ is a label of an i^{th} input value predicted by the model.

17. A device for diagnosing cancer based on immunological profiling, the device comprising:
a decoder configured to decode whole transcriptome sequencing (WTS) information from a biological sample;
an aligner configured to align the decoded WTS information to a reference genome database;
a feature detector configured to extract immune cell receptor features and transcript features from the aligned WTS information; and
a cancer diagnostic unit configured to compare the analyzed output obtained by inputting the extracted features to an artificial intelligence model trained to distinguish between a cancer patient and a normal subject, with a cut-off value, and determine whether or not cancer is present.

18. A computer-readable storage medium including an instruction configured to be executed by a processor for providing information for diagnosis of cancer, through the following steps comprising:
(a) obtaining whole transcriptome sequencing (WTS) information from a biological sample;
(b) aligning the obtained WTS information to a reference genome database;
(c) extracting immune cell receptor features and transcriptome features from the aligned WTS information;
(d) inputting the extracted features to an artificial intelligence model trained to distinguish between a cancer patient and a normal subject, comparing the analyzed output value with a cut-off value, and primarily determining whether or not there is cancer; and
(e) inputting the immune cell receptor features and transcriptome features of a sample primarily determined that there is cancer into a second artificial intelligence model trained to distinguish between an immunoactive state and cancer, comparing the analyzed output value with a cut-off value, and finally determining whether or not there is cancer.
